(19) Europäisches Patentamt
European Patent Office
Office européen des brevets

(11) Publication number: **0 306 863 B1**

(12) # EUROPEAN PATENT SPECIFICATION

(45) Date of publication of patent specification: **01.12.93**

(51) Int. Cl.5: **C07D 501/46**, A61K 31/545

(21) Application number: **88114406.7**

(22) Date of filing: **03.09.88**

The file contains technical information submitted after the application was filed and not included in this specification

(54) **Cephem compounds and processes for preparation thereof.**

(30) Priority: **07.09.87 GB 8721016**

(43) Date of publication of application:
**15.03.89 Bulletin 89/11**

(45) Publication of the grant of the patent:
**01.12.93 Bulletin 93/48**

(84) Designated Contracting States:
**AT BE CH DE ES FR GB GR IT LI LU NL SE**

(56) References cited:
**EP-A- 0 047 977**
**EP-A- 0 062 321**
**US-A- 4 521 413**

(73) Proprietor: **FUJISAWA PHARMACEUTICAL CO., LTD.**
**3, Doshomachi 4-chome**
**Higashi-ku**
**Osaka-shi Osaka 541(JP)**

(72) Inventor: **Takaya, Takao**
**5-87, Suimeidai 1-chome**
**Kawanishi-shi Hyogo 666-01(JP)**
Inventor: **Sakane, Kazuo**
**15, Azayamagata**
**Mino**
**Kawanishi-shi Hyogo 666-01(JP)**
Inventor: **Miyai, Kenzi**
**2-7-10, Higashikushiro**
**Kawanishi-shi Hyogo 666(JP)**
Inventor: **Kawabata, Kohji**
**7-2-1203, Tomobuchicho 1-chome**
**Miyakojima-ku**
**Osaka-shi Osaka 534(JP)**

(74) Representative: **Türk, Dietmar, Dr. rer. nat.**
**Türk, Gille, Hrabal, Leifert**
**Patentanwälte**
**Brucknerstrasse 20**
**D-40593 Düsseldorf (DE)**

EP 0 306 863 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel cephem compounds and pharmaceutically acceptable salts thereof. More particularly, it relates to novel cephem compounds and pharmaceutically acceptable salts thereof, which have antimicrobial activities, to processes for preparation thereof, to pharmaceutical composition comprising the same and to the use of the novel cephem compounds and pharmaceutically acceptable salts thereof for the manufacture of medicaments for treating infectious diseases in human being and animals.

From US-A-4 521 413 cephem compounds having an antimicrobial activity are already known which differ from the novel cephem compounds of the present invention of general formula (I) following below in that the cephem compounds of the present invention are substituted by a pyrazolio group, whereas the compounds of US-A-4 521 413 are substituted by a pyridinio group.

In EP-A-0 047 977 cephalosporine compounds having an antibiotic activity are known which are structurally very similar to the cephem compounds (I) of the present invention, which, however, differ from the latter ones in that the pyrazolio ring of the cephem compounds of the present invention is substituted by an amino or amino substituted group, while the cephalosporine compounds of EP-A-0 047 977 do not exhibit such substituents.

In EP-A-0 062 321 cephem compounds are disclosed having the same basic structure as the cephem compounds (I) of the present invention. The compounds of the present invention, however, can be considered to be a novel selection from the compounds disclosed in EP-A-0 062 321 in so far as (a) the heterocyclic group has been selected as being a pyrazolio group, (b) the 2-position of the pyrazolio ring is substituted by the specific group $R_3$ and (c) a selection of further substituents on the pyrazolio ring is provided. Furthermore the cephem compounds of the present invention always have at least two substituents on the pyrazolio ring which correspond to a heterocyclic cation group of EP-A-0 062 321 and one of that plural substituents on the pyrazolio ring of the invention is always amino or substituted amino.

Although the cephem compounds disclosed in EP-A-0 062 321 have antimicrobial activities, their antibacterial effect is no more sufficient for modern requirements.

Accordingly, it is one object of the present invention to provide novel cephem compounds having an improved activity against pathogenic microorganisms.

An other object of the invention is to provide processes for the preparation of these novel cephem compounds and to provide pharmaceutical compositions comprising the same as an active ingredient for treating infectious diseases caused by pathogenic microorganisms in human being and animals.

According to one aspect the present invention relates to novel cephem compounds and pharmaceutically acceptable salts thereof which have improved antimicrobial activities and can be represented by the following general formula (I):

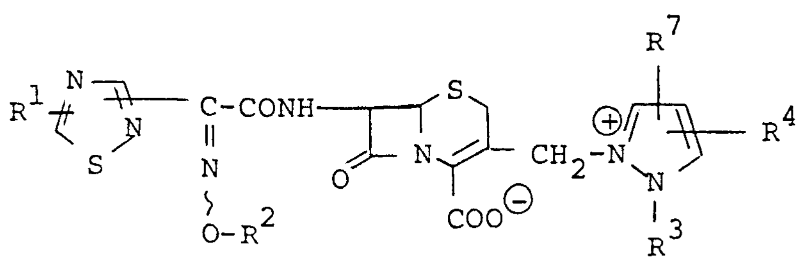 [I]

wherein

$R^1$ is amino or protected amino,

$R^2$ is $C_1$-$C_6$-alkyl or $C_1$-$C_6$-alkenyl,

$R^3$ is $C_1$-$C_6$-alkyl, hydroxy($C_1$-$C_6$)alkyl or protected hydroxy($C_1$-$C_6$)alkyl,

$R^4$ is amino, protected amino, $C_1$-$C_6$-alkylamino,proteced $C_1$-$C_6$-alkylamino, carboxy($C_1$-$C_6$)-alkylamino, N-[protected carboxy($C_1$-$C_6$)alkyl]amino, and

$R^7$ is hydrogen or $C_1$-$C_6$-alkyl.

The novel cephem compounds of the present invention have a much higher antibacterial activity than the cephem compounds disclosed in EP-A-0 062 321 as has been demonstrated by comparative tests.

Preferred embodiments of the claimed novel cephem-compounds are described in subclaims 2 to 4.

A particularly preferred cephem compound of the present invention is 7β-[2-(5-amino-1,2,4-thiadiazol-3-yl)-2-methoxyiminoacetamido]-3-(3-amino-2-methyl-1-pyrazolio)methyl-3-cephem-4-carboxylate (syn isomer).

As to the novel cephem compounds of formula (I) the following points are to be noted:
That is, the object compound [I] includes syn isomer, anti isomer and a mixture thereof. Syn isomer means one geometrical isomer having the partial structure represented by the following formula:

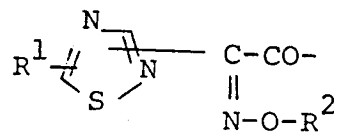

(wherein $R^1$ and $R^2$ are each as defined above) and anti isomer means the other geometrical isomer having the partial structure represented by the following formula:

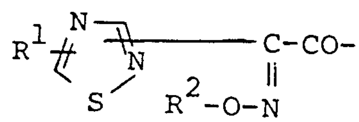

(wherein $R^1$ and $R^2$ are each as defined above), and all of such geometrical isomers and mixture thereof are included within the scope of this invention.

In the present specification and claim the partial structure of these geometrical isomers and mixture thereof are represented for convenient sake by the following formula:

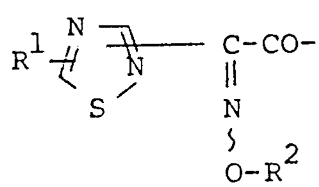

(wherein $R^1$ and $R^2$ are each as defined above).

Another point to be noted is that the pyrazolio moiety of the compound [I] can also exist in the tautomeric form, and such tautomeric equilibrium can be represented by the following schemes.

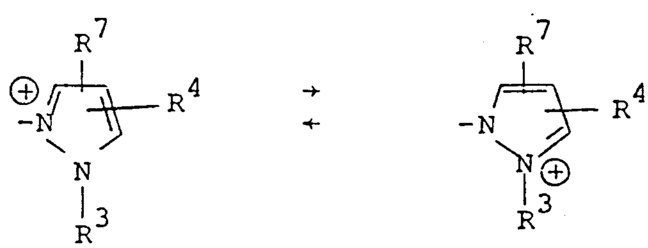

(A) (B)

(wherein $R^3$, $R^4$ and $R^7$ are each as defined above).

Both of the above tautomeric isomers are included within the scope of the present invention, and in the present specification and claim, however, the object compound [I] is represented for the convenient sake by one expression of the pyrazolio group of the formula (A).

According to a second aspect the present invention relates to a process for preparing the novel cephem compounds of general formula (I) and salts thereof, which comprises

(1) reacting a compound of the formula :

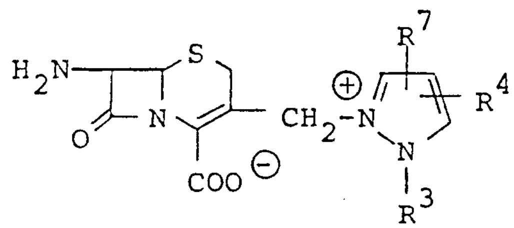

wherein $R^3$, $R^4$ and $R^7$ are each as defined above, or its reactive derivative at the amino group or a salt thereof with a compound of the formula :

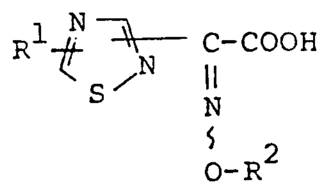

wherein $R^1$ and $R^2$ are each as defined above, or its reactive derivative at the carboxy group or a salt thereof to give a compound of the formula :

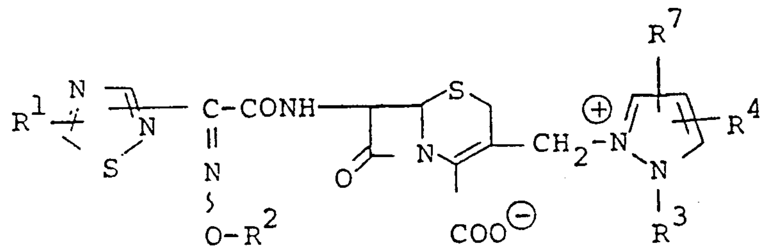

wherein

$R^1$, $R^2$, $R^3$, $R^4$ and $R^7$ are each as defined above,

or a salt thereof, or

(2) subjecting a compound of the formula :

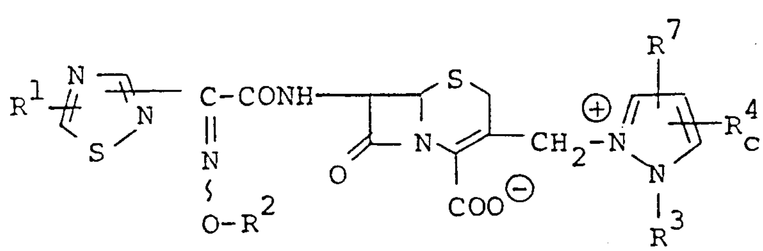

wherein

$R^1$, $R^2$, $R^3$ and $R^7$ are each as defined above,

$R^4_c$ is N-[protected carboxy($C_1$-$C_6$)alkyl]amino,

or a salt thereof to elimination reaction of the carboxy protective group to give a compound of the formula :

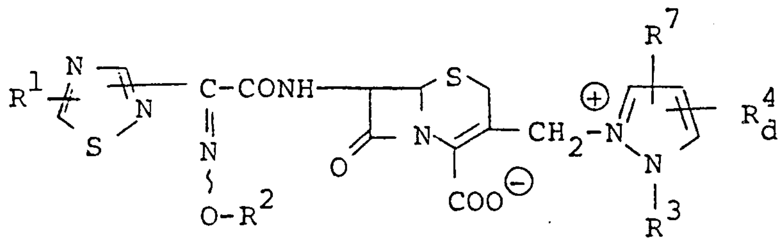

wherein

$R^1$, $R^2$, $R^3$ and $R^7$ are each as defined above,

$R^4_d$ is carboxy($C_1$-$C_6$)alkylamino,

or a salt thereof, or

(3) subjecting a compound of the formula :

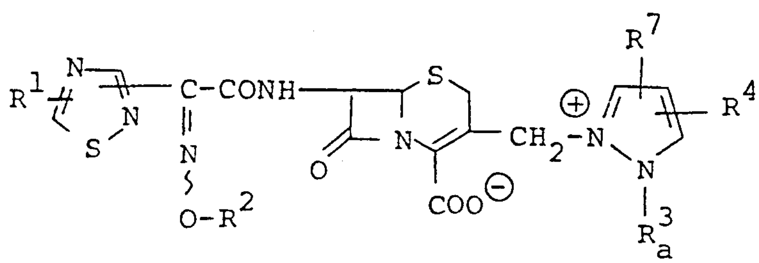

wherein

$R^1$, $R^2$, $R^4$ and $R^7$ are each as defined above,

$R^3_a$ is protected hydroxy($C_1$-$C_6$)alkyl,

or a salt thereof to elimination reaction of the hydroxy protective group to give a compound of the formula :

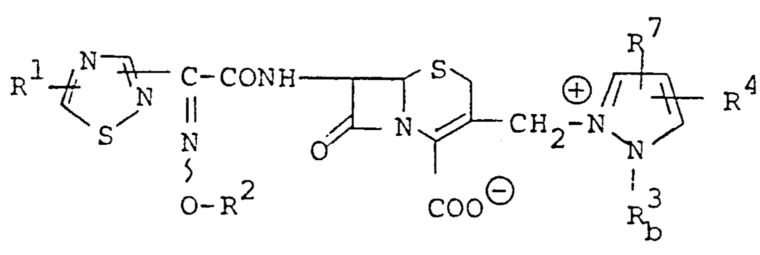

wherein

$R^1$, $R^2$, $R^4$ and $R^7$ are each as defined above,

$R^3_b$ is hydroxy($C_1$-$C_6$)alkyl,

or a salt thereof.

According to a third aspect the present invention relates to a pharmaceutical composition which comprises as an active ingredient a cephem compound of formula (I) or a pharmaceutically acceptable salt thereof in admixture with pharmaceutically acceptable carriers.

Furthermore, the present invention relates to the use of the novel cephem compounds of formula (I) or pharmaceutically acceptable salts thereof for the manufacture of medicaments for treating infectious diseases caused by pathogenic microorganisms in human being and animals.

The cephem compound [I] of the present invention can be prepared by processes as illustrated in the following.

Process 1

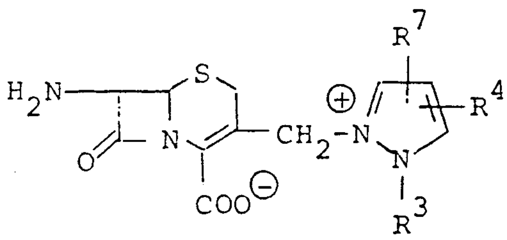

+

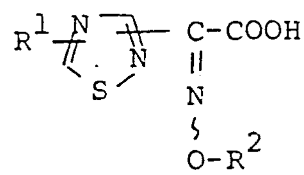

[II]

or its reactive derivative at the amino group or a salt thereof

[III]

or its reactive derivative at the carboxy group or a salt thereof

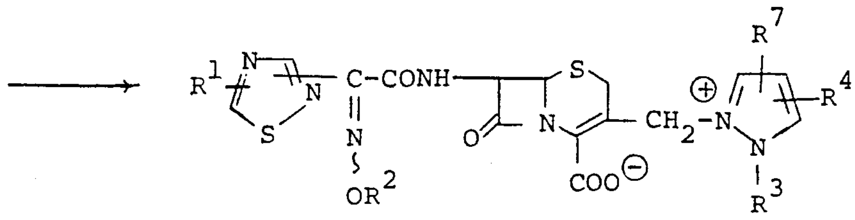

[I]

or a salt thereof

Process 2

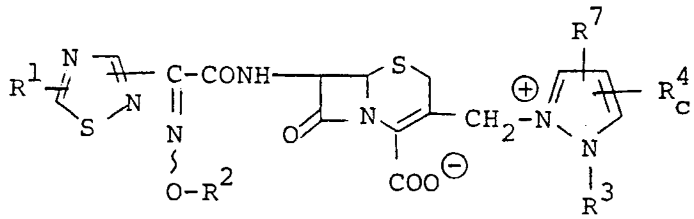

[Ia]

or a salt thereof

Elimination reaction of the
carboxy protective group

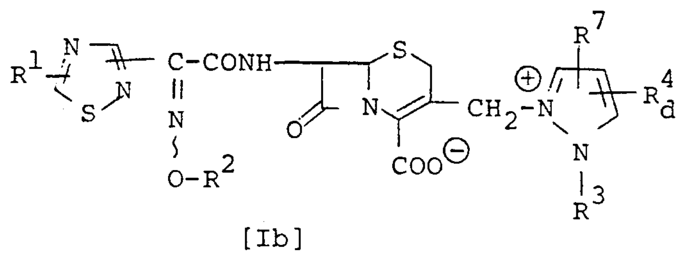

[Ib]

or a salt thereof

Process 3

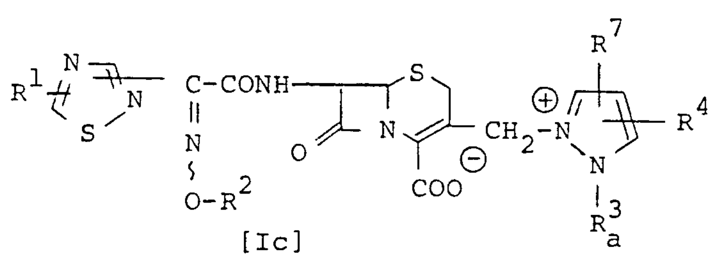

[Ic]

or a salt thereof

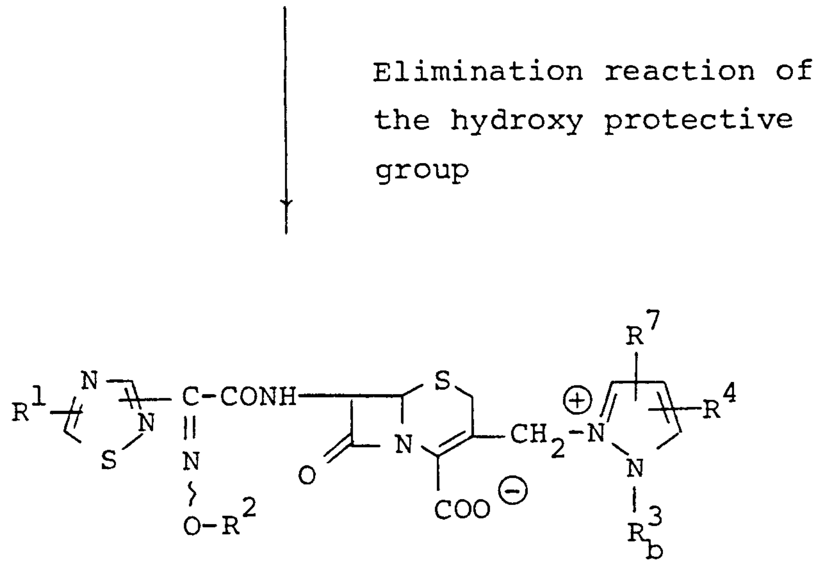


[Id]

or a salt thereof

wherein

| | |
|---|---|
| $R^1$, $R^2$, $R^3$, $R^4$ and $R^7$ | are each as defined above, |
| $R_c^4$ is | N-[protected carboxy($C_1$-$C_6$)alkyl]amino, |
| $R_d^4$ is | carboxy($C_1$-$C_6$)alkylamino, |
| $R_a^3$ is | protected hydroxy($C_1$-$C_6$)alkyl, and |
| $R_b^3$ is | hydroxy($C_1$-$C_6$)alkyl. |

The starting compound [II] can be prepared by the following processes.

Process A

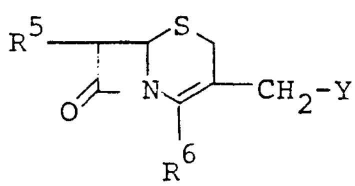

[IV]

or a salt thereof

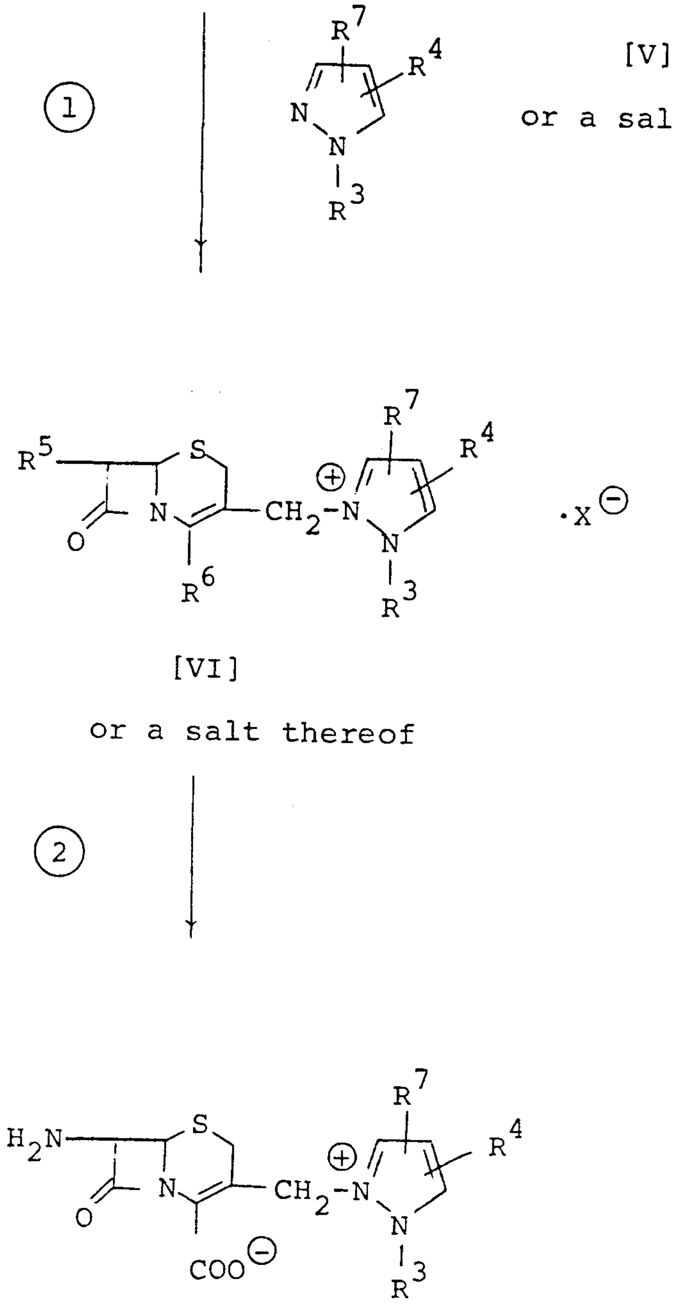


[V]

or a salt thereof

[VI]

or a salt thereof

[II]

or a salt thereof

Process B

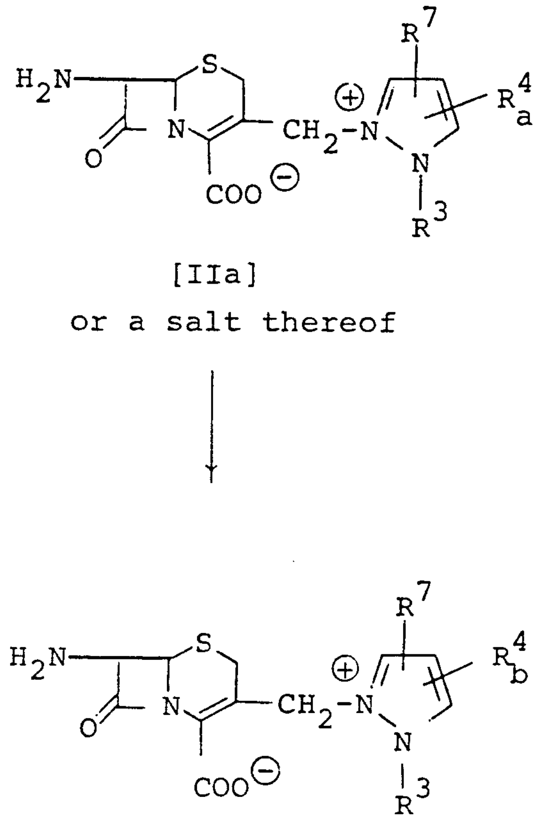

[IIa]

or a salt thereof

[IIb]

or a salt thereof

wherein

| | |
|---|---|
| $R^3$, $R^4$ and $R^7$ | are each as defined above, |
| $R^5$ is | protected amino, |
| $R^6$ is | protected carboxy, |
| Y is | a leaving group, |
| $X^{\ominus}$ is | an anion, |
| $R^4_a$ is | protected amino or protected $C_1$-$C_6$-alkylamino, and |
| $R^4_b$ is | amino or $C_1$-$C_6$-alkylamino. |

The starting compound [V] or a salt thereof can be prepared by the methods disclosed in the Preparations 1 to 8 described later or similar manners thereto.

In the above and subsequent descriptions of this specification, suitable examples of the various definitions are explained in detail as follows :

Suitable "amino protective group" in the "protected amino" and "protected $C_1$-$C_6$-alkylamino" may be an acyl group as mentioned below, substituted or unsubstituted ar($C_1$-$C_6$)alkylidene [e.g. benzylidene, and hydroxybenzylidene,], ar($C_1$-$C_6$)alkyl such as mono or di or triphenyl($C_1$-$C_6$)alkyl [e.g. benzyl, phenethyl, benzhydryl, and trityl].

Suitable "acyl" may be $C_1$-$C_6$-alkanoyl [e.g. formyl, acetyl, propionyl, hexanoyl, and pivaloyl], mono(or di or tri)halo($C_1$-$C_6$)alkanoyl [e.g. chloroacetyl, and trifluoroacetyl.], $C_1$-$C_6$-alkoxycarbonyl [e.g. methoxycarbonyl, ethoxycarbonyl, tert-butoxycarbonyl, tert-pentyloxycarbonyl, and hexyloxycarbonyl.], carbamoyl, aroyl [e.g. benzoyl, toluoyl, and naphthoyl], ar($C_1$-$C_6$)alkanoyl [e.g. phenylacetyl, and phenylpropionyl,], aryloxycarbonyl [e.g. phenoxycarbonyl, and naphthyloxycarbonyl], aryloxy($C_1$-$C_6$)alkanoyl [e.g. phenoxyacetyl,

and phenoxypropionyl], arylglyoxyloyl [e.g. phenylglyoxyloyl, and naphthylglyoxyloyl,], ar($C_1$-$C_6$)-alkoxycarbonyl which may have suitable substituent(s) [e.g. benzyloxycarbonyl, phenethyloxycarbonyl, and p-nitrobenzyloxycarbonyl]

Suitable "$C_1$-$C_6$-alkyl" and "$C_1$-$C_6$-alkyl moieties" in the "hydroxy($C_1$-$C_6$)alkyl", "protected hydroxy($C_1$-$C_6$)alkyl", "$C_1$-$C_6$-alkylamino", "protected $C_1$-$C_6$-alkylamino", "carboxy($C_1$-$C_6$)alkylamino" and "N-[protected carboxy($C_1$-$C_6$)alkyl]amino", may be a straight or branched one such as methyl, ethyl, propyl, isopropyl, butyl, isobutyl, tert-butyl, pentyl, or hexyl.

Suitable "$C_1$-$C_6$-alkenyl" may be a straight or branched one such as vinyl, allyl, 1-propenyl, isopropenyl, butenyl, isobutenyl, pentenyl, or hexenyl.

Suitable "protected hydroxy" in the "protected hydroxy ($C_1$-$C_6$)-alkyl" may be acyloxy group. Suitable "acyl moiety" in the "acyloxy" may be $C_1$-$C_6$-alkanoyl [e.g. formyl, acetyl, propionyl, hexanoyl, and pivaloyl], mono(or di or tri)halo($C_1$-$C_6$)alkanoyl [e.g. chloroacetyl, and trifluoroacetyl], $C_1$-$C_6$-alkoxycarbonyl [e.g. methoxycarbonyl, ethoxycarbonyl, tert-butoxycarbonyl, tert-pentyloxycarbonyl, and hexyloxycarbonyl], or carbamoyl.

Suitable "protected carboxy" and "protected carboxy moiety" in the term "N-[protected carboxy($C_1$-$C_6$)alkyl]amino" may be an esterified carboxy group, and concrete examples of the ester moiety in said esterified carboxy group may be the ones such as $C_1$-$C_6$-alkyl ester [e.g. methyl ester, ethyl ester, propyl ester, isopropyl ester, butyl ester, isobutyl ester, tert-butyl ester, pentyl ester, hexyl ester, and 1-cyclopropylethyl ester] which may have suitable substituent(s), for example, $C_1$-$C_6$-alkanoyloxy($C_1$-$C_6$)alkyl ester [e.g. acetoxymethyl ester, propionyloxymethyl ester, butyryloxymethyl ester, valeryloxymethyl ester, pivaloyloxymethyl ester, 1-acetoxyethyl ester, 1-propionyloxyethyl ester, pivaloyloxymethyl ester, 2-propionyoxyethyl ester, and hexanoyloxymethyl ester,], $C_1$-$C_6$-alkanesulfonyl($C_1$-$C_6$)alkyl ester [e.g. 2-mesylethyl ester] or mono(or di or tri)halo($C_1$-$C_6$)alkyl ester [e.g. 2-iodoethyl ester, 2,2,2-trichloroethyl ester]; $C_1$-$C_6$-alkenyl ester [e.g. vinyl ester, allyl ester]; $C_1$-$C_6$-alkynyl ester (e.g. ethynyl ester, and propynyl ester]; ar($C_1$-$C_6$)alkyl ester which may have suitable substituent(s) [e.g. benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, and 4-hydroxy-3,5-di-tert-butylbenzyl ester]; aryl ester which may have suitable substituent(s) [e.g. phenyl ester, 4-chlorophenyl ester, tolyl ester, 4-tert-butylphenyl ester, xylyl ester, mesityl ester, and cumenyl ester].

Suitable "leaving group" may be halogen [e.g. chlorine, bromine, and iodine], acyloxy such as sulfonyloxy [e.g. benzenesulfonyloxy, tosyloxy, and mesyloxy], $C_1$-$C_6$-alkanoyloxy [e.g. acetyloxy, and propionyloxy].

Suitable "anion" may be formate, acetate, trifluoroacetate, maleate, tartrate, methanesulfonate, benzenesulfonate, toluenesulfonate, chloride, bromide, iodide, sulfate, or phosphate.

Suitable pharmaceutically acceptable salts of the object compound [I] are conventional non-toxic salts and include a metal salt such as an alkali metal salt [e.g. sodium salt, and potassium salt and an alkaline earth metal salt [e.g. calcium salt, and magnesium salt,], an ammonium salt, an organic base salt [e.g. trimethylamine salt, triethylamine salt, pyridine salt, picoline salt, dicyclohexylamine salt, and N,N'-dibenzylethylenediamine salt], an organic acid salt [e.g. formate, acetate, trifluoroacetate, maleate, tartrate, methanesulfonate, benzenesulfonate, and toluenesulfonate,], an inorganic acid salt [e.g. hydrochloride, hydrobromide, sulfate, and phosphate], a salt with an amino acid [e.g. arginine salt, aspartic acid salt, and glutamic acid salt].

Preferred embodiments of the object compound [I] are as follows.

Preferred embodiment of $R^1$ is amino,

$R^2$ is $C_1$-$C_6$-alkyl [more preferably ($C_1$-$C_4$)alkyl] or $C_1$-$C_6$-alkenyl [more preferably ($C_2$-$C_4$)alkenyl, most preferably propenyl],

$R^3$ is $C_1$-$C_6$-alkyl [more preferably($C_1$-$C_4$)alkyl, most preferably methyl], hydroxy($C_1$-$C_6$)alkyl [more preferably hydroxy($C_1$-$C_4$)alkyl, most preferably hydroxyethyl] or acyloxy($C_1$-$C_6$)alkyl [more preferably carbamoyloxy($C_1$-$C_6$)alkyl or $C_1$-$C_6$-alkanoyloxy($C_1$-$C_6$)alkyl, most preferably carbamoyloxy($C_1$-$C_4$)alkyl or $C_1$-$C_6$-alkanoyloxy($C_1$-$C_4$)alkyl],

$R^4$ is amino, acylamino [more preferably carbamoylamino or $C_1$-$C_6$-alkanoylamino], $C_1$-$C_6$-alkylamino, carboxy($C_1$-$C_6$)alkylamino or N-[protected carboxy($C_1$-$C_6$)alkyl]amino [more preferably esterified carboxy($C_1$-$C_6$)alkylamino,most preferably $C_1$-$C_6$-alkoxycarbonyl($C_1$-$C_6$)-alkylamino],

$R^7$ is hydrogen or $C_1$-$C_6$-alkyl [more preferably ($C_1$-$C_4$)alkyl, most preferably methyl].

The processes for preparing the object compound of the present invention are explained in detail in the following.

Process 1

The compound [I] or a salt thereof can be prepared by reacting the compound [II] or its reactive derivative at the amino group or a salt thereof with the compound [III] or its reactive derivative at the carboxy group or a salt thereof.

Suitable reactive derivative at the amino group of the compound [II] may include Schiff's base type imino or its tautomeric enamine type isomer formed by the reaction of the compound [II] with a carbonyl compound such as aldehyde, or ketone; a silyl derivative formed by the reaction of the compound [II] with a silyl compound such as bis(trimethylsilyl)acetamide, mono(trimethylsilyl)acetamide [e.g. N-(trimethylsilyl)-acetamide], or bis(trimethylsilyl)urea;
a derivative formed by reaction of the compound [II] with phosphorus trichloride or phosgene.

Suitable salts of the compound [II] and its reactive derivative can be referred to the ones as exemplified for the compound [I].

Suitable reactive derivative at the carboxy group of the compound [III] may include an acid halide, an acid anhydride, an activated amide, and an activated ester. Suitable examples of the reactive derivatives may be an acid chloride; an acid azide; a mixed acid anhydride with an acid such as substituted phosphoric acid [e.g. dialkylphosphoric acid, phenylphosphoric acid, diphenylphosphoric acid, dibenzylphosphoric acid, and halogenated phosphoric acid,], dialkylphosphorous acid, sulfurous acid, thiosulfuric acid, sulfuric acid, sulfonic acid [e.g. methanesulfonic acid], aliphatic carboxylic acid [e.g. acetic acid, propionic acid, butyric acid, isobutyric acid, pivalic acid, pentanoic acid, isopentanoic acid, 2-ethylbutyric acid, and trichloroacetic acid] or aromatic carboxylic acid [e.g. benzoic acid]; a symmetrical acid anhydride; an activated amide with imidazole, 4-substituted imidazole, dimethylpyrazole, triazole or tetrazole; or an activated ester [e.g. cyanomethyl ester, methoxymethyl ester, dimethyliminomethyl $[(CH_3)_2\overset{+}{N}=CH-]$ ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, and 8-quinolyl thioester], or an ester with a N-hydroxy compound [e.g. N,N-dimethylhydroxylamine, 1-hydroxy-2-(1H)-pyridone, N-hydroxysuccinimide, N-hydroxyphthalimide, and 1-hydroxy-1H-benzotriazole]. These reactive derivatives can optionally be selected from them according to the kind of the compound [III] to be used.

Suitable salts of the compound [III] and its reactive derivative can be referred to the ones as exemplified for the compound [I].

The reaction is usually carried out in a conventional solvent such as water, alcohol [e.g. methanol, and ethanol,], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.

In this reaction, when the compound [III] is used in a free acid form or its salt form, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)-carbodiimide; N,N'-diethylcarbodiimide, N,N'-diisopropylcarbodiimide; N-ethyl-N'-(3-dimethylaminopropyl)-carbodiimide; N,N'-carbonylbis-(2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; thionyl chloride; oxalyl chloride; $C_1$-$C_6$ alkyl haloformate [e.g. ethyl chloroformate, and isopropyl chloroformate]; triphenylphosphine; 2-ethyl-7-hydroxybenzisoxazolium salt; 2-ethyl-5-(m-sulfophenyl)-isoxazolium hydroxide intramolecular salt; 1-(p-chlorobenzenesulfonyloxy)-6-chloro-1H-benzotriazole; so-called Vilsmeier reagent prepared by the reaction of N,N-dimethylformamide with thionyl chloride, phosgene, trichloromethyl chloroformate, and phosphorus oxychloride,.

The reaction may also be carried out in the presence of an inorganic or organic base such as an alkali metal bicarbonate, tri(lower)alkylamine, pyridine, N-($C_1$-$_6$)alkylmorpholine, or N,N-di($C_1$-$_6$)alkylbenzylamine.

The reaction temperature is not critical, and the reaction is usually carried out under cooling to warming.

Process 2

The compound [Ib] or a salt thereof can be prepared by subjecting the compound [Ia] or a salt thereof to elimination reaction of the carboxy protective group.

Suitable method of this elimination reaction may include conventional one such as hydrolysis, and reduction.

(i) For Hydrolysis :

The hydrolysis is preferably carried out in the presence of a base or an acid including Lewis acid. Suitable base may include an inorganic base and an organic base such as an alkali metal [e.g. sodium, and potassium], an alkaline earth metal [e.g. magnesium, and calcium], the hydroxide or carbonate or bicarbonate thereof, trialkylamine [e.g. trimethylamine, and triethylamine,], picoline, 1,5-diazabicyclo[4.3.0]-non-5-ene, 1,4-diazabicyclo[2.2.2]octane, or 1,8-diazabicyclo[5.4.0]undec-7-ene.

Suitable acid may include an organic acid [e.g. formic acid, acetic acid, propionic acid, trichloroacetic acid, and trifluoroacetic acid] and an inorganic acid [e.g. hydrochloric acid, hydrobromic acid, sulfuric acid, hydrogen chloride, and hydrogen bromide].
The elimination using Lewis acid such as trihaloacetic acid [e.g. trichloroacetic acid, and trifluoroacetic acid, ] is preferably carried out in the presence of cation trapping agents [e.g. anisole, and phenol].

The reaction is usually carried out in a solvent such as water, an alcohol [e.g. methanol, and ethanol], methylene chloride, tetrahydrofuran, a mixture thereof or any other solvent which does not adversely influence the reaction. A liquid base or acid can be also used as the solvent. The reaction temperature is not critical and the reaction is usually carried out under cooling to warming.

(ii) For Reduction :

Reduction is carried out in a conventional manner, including chemical reduction and catalytic reduction.

Suitable reducing agents to be used in chemical reduction are a combination of a metal (e.g. tin, zinc, and iron) or metallic compound (e.g. chromium chloride, and chromium acetate) and an organic or inorganic acid (e.g. formic acid, acetic acid, propionic acid, trifluoroacetic acid, p-toluenesulfonic acid, hydrochloric acid, and hydrobromic acid).

Suitable catalysts to be used in catalytic reduction are conventional ones such as platinum catalysts (e.g. platinum plate, spongy platinum, platinum black, colloidal platinum, platinum oxide, and platinum wire), palladium catalysts (e.g. spongy palladium, palladium black, palladium oxide, palladium on carbon, colloidal palladium, palladium on barium sulfate, and palladium on barium carbonate), nickel catalysts (e.g. reduced nickel, nickel oxide, and Raney nickel), cobalt catalysts (e.g. reduced cobalt and Raney cobalt), iron catalysts (e.g. reduced iron, and Raney iron), copper catalysts (e.g. reduced copper Raney copper, and Ullman copper. The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran, or a mixture thereof.

The reaction temperature of this reduction is not critical and the reaction is usually carried out under cooling to warming.

Process 3

The compound [Id] or a salt thereof can be prepared by subjecting the compound [Ic] or a salt thereof to elimination reaction of the hydroxy protective group. This reaction can be carried out in a similar manner to that of the aforementioned Process 2, and therefore the reagents to be used and the reaction conditions (e.g., solvent, and reaction temperature.) can be referred to those of the Process 2.

Processes A and B for the preparation of the starting compounds are explained in detail in the following.

Process A - ①

The compound [VI] or a salt thereof can be prepared by reacting the compound [IV] or a salt thereof with the compound [V] or a salt thereof.

Suitable salts of the compounds [V] and [VI] can be referred to the ones as exemplified for the compound [I].

The present reaction may be carried out in a solvent such as water, phosphate buffer, acetone, chloroform, acetonitrile, nitrobenzene, methylene chloride, ethylene chloride, formamide, N,N-dimethylformamide, methanol, ethanol, diethyl ether, tetrahydrofuran, dimethyl sulfoxide, or any other organic solvent which does not adversely affect the reaction, preferably in ones having strong polarities. Among the solvents, hydrophilic solvents may be used in a mixture with water. When the compound [V] is in liquid, it

can also be used as a solvent. The reaction is preferably conducted in the presence of a base, for example, inorganic base such as alkali metal hydroxide, alkali metal carbonate, alkali metal bicarbonate, organic base such as trialkylamine. The reaction temperature is not critical, and the reaction is usually carried out at ambient temperature, under warming or under heating. The present reaction is preferably carried out in the presence of alkali metal halide [e.g. sodium iodide, and potassium iodide], alkali metal thiocyanate [e.g. sodium thiocyanate, and potassium thiocyanate].

Anion $X^{\ominus}$ may be the one derived from a leaving group Y and may be the other one converted therefrom by a conventional method.

Process A - ②

The compound [II] or a salt thereof can be prepared by subjecting the compound [VI] or a salt thereof to elimination reaction of the amino protective group in $R^5$ and the carboxy protective group in $R^6$.

This reaction is carried out in accordance with a conventional method such as hydrolysis.

The hydrolysis is preferably carried out in the presence of a base or an acid including Lewis acid. Suitable base may include an inorganic base and an organic base such as an alkali metal [e.g. sodium, and potassium], an alkaline earth metal [e.g. magnesium, and calcium], the hydroxide or carbonate or bicarbonate thereof, trialkylamine [e.g. trimethylamine, and triethylamine], picoline, 1,5-diazabicyclo[4.3.0]-non-5-ene, 1,4-diazabicyclo[2.2.2]octane, or 1,8-diazabicyclo[5.4.0]undec-7-ene.

Suitable acid may include an organic acid [e.g. formic acid, acetic acid, propionic acid, trichloroacetic acid, and trifluoroacetic acid] and an inorganic acid [e.g. hydrochloric acid, hydrobromic acid, sulfuric acid,, hydrogen chloride, and hydrogen bromide].

The elimination using Lewis acid such as trihaloacetic acid [e.g. trichloroacetic acid, and trifluoroacetic acid, ] is preferably carried out in the presence of cation trapping agents [e.g. anisole, and phenol].

The reaction is usually carried out in a solvent such as water, an alcohol [e.g. methanol, and ethanol], methylene chloride, tetrahydrofuran, a mixture thereof or any other solvent which does not adversely influence the reaction. A liquid base or acid can be also used as the solvent. The reaction temperature is not critical and the reaction is usually carried out under cooling to warming.

The present invention includes within the scope of the invention the case that protected amino in $R^4$ is transformed into amino during this reaction.

Process B

The compound [IIb] or a salt thereof can be prepared by subjecting the compound [IIa] to elimination reaction of the amino protective group in $R^4_a$ . This reaction can be carried out in a similar manner to that of the aforementioned Process A - ② , and therefore the reagents to be used and the reaction conditions (e.g., solvent, and reaction temperature.) can be referred to those of the Process A - ② .

The present invention includes within the scope of the invention the case that protected hydroxy($C_{1-6}$)alkyl in $R^3$ is transformed into hydroxy($C_{1-6}$)alkyl during this reaction.

The object compound [I] and pharmaceutically acceptable salts thereof are novel and exhibit high antimicrobial activity, inhibiting the growth of a wide variety of pathogenic microorganisms including Gram-positive and Gram-negative microorganisms and are useful as antimicrobial agents.

Now in order to show the utility of the object compound [I], the test data on MIC (minimal inhibitory concentration) of a representative compound of this invention are shown in-the following.

Test method :

In vitro antibacterial activity was determined by the two-fold agar-plate dilution method as described below.

One loopful of an overnight culture of each test strain in Trypticase-soy broth ($10^6$ viable cells per ml) was streaked on heart infusion agar (HI-agar) containing graded concentrations of representative test compound, and the minimal inhibitory concentration (MIC) was expressed in terms of µg/mℓ after incubation at 37 °C for 20 hours.

Test compound :

7β-[2-(5-Amino-1,2,4-thiadiazol-3-yl)-2-methoxy-iminoacetamido]-3-(3-amino-2-methyl-1-pyrazolio)-methyl-3-cephem-4-carboxylate (syn isomer) (hereinafter referred to as Compound A)

Test results :

| Test strain | MIC (μg/mℓ) Test compound |
|---|---|
| | A |
| E. coli 31 | 0.05 |

For therapeutic administration, the object compound [I] and pharmaceutically acceptable salts thereof of the present invention are used in the form of conventional pharmaceutical preparation which contains said compound as an active ingredient, in admixture with pharmaceutically acceptable carriers such as an organic or inorganic solid or liquid excipient which is suitable for oral, parenteral and external administration. The pharmaceutical preparations may be in solid form such as tablet, granule, powder, capsule, or liquid form such as solution, suspension, syrup, emulsion, and lemonade .

If needed, there may be included in the above preparations auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter, and ethylene glycol.

While the dosage of the compound [I] may vary from and also depend upon the age, conditions of the patient, a kind of diseases, and a kind of the compound [I] to be applied. In general amounts between 1 mg and about 4,000 mg or even more per day may be administered to a patient. An average single dose of about 50 mg, 100 mg, 250 mg, 500 mg, 1000 mg, 2000 mg of the object compounds [I] of the present invention may be used in treating diseases infected by pathogenic microorganisms.

The following Preparations and Examples are given for the purpose of illustrating the present invention in more detail.

Preparation 1

(1) A mixture of acetic anhydride (11.13 ml) and formic acid (5.93 ml) was stirred at ambient temperature for 30 minutes. To this solution was added 5-amino-1-(2-hydroxyethyl)pyrazole (5 g) under ice-cooling, and the mixture was stirred at 30-40 °C for 1 hour. The reaction mixture was poured into a mixture of water, tetrahydrofuran and ethyl acetate and adjusted to pH 6 with aqueous sodium bicarbonate. The organic layer was separated, and the aqueous layer was extracted with a mixture of tetrahydrofuran and ethyl acetate for three times. The organic layers were combined, dried over magnesium sulfate and evaporated in vacuo to give 5-formamido-1-(2-formyloxyethyl)pyrazole (5.18 g).

IR (Nujol) : 3180, 1705, 1660 $cm^{-1}$

NMR (DMSO-$d_6$, δ) : 4.21-4.61 (4H, m), 6.11 and 6.34 (1H, each d, J = 3Hz), 7.47 (1H, d, J = 3Hz), 8.00 (1H, s), 8.33 (1H, s)

The following compound was obtained according to a similar manner to that of Preparation 1(1).

(2) 5-Formamido-1-(2-formyloxyethyl)-4-methylpyrazole

IR (Nujol) : 3180, 1715, 1660 $cm^{-1}$

NMR (DMSO-$d_6$, δ) : 1.81 and 1.86 (3H, each s), 4.01-4.48 (4H, m), 7.25 and 7.40 (1H, each s), 8.06 (1H, s), 8.22 and 9.13 (1H, each s)

Preparation 2

5-Amino-1-(2-hydroxyethyl)pyrazole (10 g) was added to a mixture of acetic acid (50 ml) and water (100 ml). A solution of potassium cyanate (25.5 g) in water (80 ml) was added dropwise thereto under stirring at 34 °C. The mixture was stirred at room temperature overnight.

The reaction mixture was added to ethyl acetate (200 ml). The mixture was adjusted to pH 8.0 with potassium carbonate and extracted with tetrahydrofuran. The extract was dried over magnesium sulfate, and the organic solvent was evaporated in vacuo to give an oily product. Acetone was added to the said oily product to give 5-carbamoylamino-1-(2-hydroxyethyl)pyrazole (3.76 g) as amorphous solid.

IR (Nujol) : 3400, 3200, 1670, 1570 $cm^{-1}$

NMR (DMSO-$d_6$, δ) : 3.64 (2H, t, J = 6Hz), 3.97 (2H, t, J = 6Hz), 6.02 and 6.13 (1H, each d, J = 2Hz), 7.20 and 7.27 (1H, each d, J = 2Hz)

Preparation 3

Formic acid (2.09 ml) was added to acetic anhydride (4.17 ml) at room temperature and the mixture was stirred for 30 minutes at the same temperature. 5-Carbamoylamino-1-(2-hydroxyethyl)pyrazole (3.76 g) was added thereto under stirring and ice-cooling. The mixture was stirred for 2 hours at room temperature. The reaction mixture was evaporated in vacuo and diisopropyl ether was added thereto to give 5-carbamoylamino-1-(2-formyloxyethyl)pyrazole (4.15 g).

mp : 102-104 °C

IR (Nujol) : 3400, 3200, 1710, 1660, 1560, 1170 $cm^{-1}$

NMR ($D_2O$, $\delta$) : 4.20 (2H, t, J=5Hz), 4.36 (2H, t, J=5Hz), 5.96 (1H, s), 6.02 and 6.13 (1H, each d, J=2Hz), 7.24 and 7.32 (1H, each d, J=2Hz) 8.06 and 8.24 (1H, each s)

Preparation 4

1-(2-Hydroxyethyl)-5-aminopyrazole (5 g) was added to acetic anhydride (14.7 ml) under stirring and ice-cooling, and pyridine (6.3 ml) was added thereto. The mixture was stirred for 2 hours at 25 °C. The reaction mixture was added to a mixture of ethyl acetate (50 ml) and brine (50 ml). Then, the mixture was adjusted to pH 7.0 with an aqueous solution of sodium bicarbonate. The aqueous layer was extracted with a mixture of ethyl acetate and tetrahydrofuran. The extract was dried over magnesium sulfate. The magnesium sulfate was filtered off, and the filtrate was evaporated under reduced pressure to give 1-(2-acetoxyethyl)-5-acetylaminopyrazole (5.98 g).

mp : 83-84 °C

IR (Nujol) : 3270, 1750, 1670, 1565 $cm^{-1}$

NMR (DMSO-$d_6$, $\delta$) : 1.93 (3H, s), 2.03 (3H, s), 4.22 (4H, br s), 6.13 (1H, d, J=2Hz), 7.32 (1H, d, J=2Hz), 9.76 (1H, s)

Preparation 5

A mixture of acetic anhydride (44.5 ml) and formic acid (22.3 ml) was stirred at ambient temperature for an hour. To this mixture was added 1-(2-hydroxyethyl)-5-aminopyrazole (30 g) at 0 - 10 °C, and the mixture was stirred under ice-cooling for 30 minutes. The mixture was poured into ice-cold water, adjusted to pH 10.5 with 40% aqueous potassium carbonate, and stirred under ice-cooling for 30 minutes. The mixture was extracted with a mixture of tetrahydrofuran and ethyl acetate 6 times. The organic layer was dried over magnesium sulfate and evaporated in vacuo to give 1-(2-hydroxyethyl)-5-formamidopyrazole (30.8 g).

IR (Nujol) : 3230, 1695, 1570, 1540 $cm^{-1}$

NMR (DMSO-$d_6$, $\delta$) : 3.62-3.95 (2H, m), 3.98-4.32 (2H, m), 6.22 and 6.36 (1H, each d, J=3Hz), 7.42 (1H, d, J=3Hz), 8.32 and 8.36 (1H, each s)

Preparation 6

To a suspension of 5-formamido-1-(2-hydroxyethyl)pyrazole (1 g) in acetonitrile (50 ml) was added dropwise chlorosulfonyl isocyanate (0.77 ml) at -15 °C ∿ - 20 °C. The mixture was stirred for 3 hours under ice-cooling. To the reaction mixture was added water (1 ml) and kept to stand overnight. The solution was adjusted to pH 7.5 with 5N-sodium hydroxide solution and then adjusted to pH 8.5 with 1N-sodium hydroxide solution. The organic layer was separated and the aqueous layer was extracted with tetrahydrofuran. The extract and said organic layer were combined and dried over magnesium sulfate. The solvent was distilled off and the residue was crystallized from ethyl acetate to give 5-amino-1-(2-carbamoyloxyethyl)pyrazole (0.60 g).

NMR (DMSO-$d_6$, $\delta$) : 3.83-4.35 (4H, m), 4.80-5.18 (2H, broad s), 5.32 (1H, d, J=3Hz), 6.33-6.87 (2H, broad s), 7.08 (1H, d, J=3Hz)

Preparation 7

5-Formamido-1-(2-carbamoyloxyethyl)pyrazole (3.69 g) was obtained from 5-amino-1-(2-carbamoyloxyethyl)pyrazole (3.3 g) according to a similar manner to that of

Preparation 5.

NMR (DMSO-$d_6$, $\delta$) : 4.22 (4H, s), 6.17-6.40 (1H, m), 6.40-6.63 (2H, m), 7.30-7.53 (1H, m), 8.13-8.47 (1H, m).

Preparation 8

(1) To a solution of 5-formamido-1-methylpyrazole in N,N-dimethylformamide (50 ml) was sodium hydride (1.6 g) under ice-cooling. Then, to the mixture was added methyl iodide (2.5 ml) at the same condition. The mixture was stirred for 1 hour under ice-cooling. To the reaction mixture was added a mixture of ethyl acetate (500 ml) and water (100 ml). The organic layer was separated, and the aqueous layer was extracted with ethyl acetate (50 ml x 2). The organic solution was dried over magnesium sulfate. The solvent was distilled off and the residue was subjected to a column chromatography on silica gel using a mixture of ethyl acetate and diisopropyl ether (3:1) as an eluent. Fractions containing the object compound were collected and evaporated in vacuo to give 5-(N-formyl-N-methylamino)-1-methylpyrazole (2.5 g).

IR (Nujol) : 1660-1680, 1550, 1320 $cm^{-1}$

NMR (DMSO-$d_6$, $\delta$) : 3.07 (3H, s), 3.67 (3H, s), 6.28 (1H, d, J=2Hz), 7.44 (1H, d, J=2Hz), 8.20 (1H, s)

The following compound was obtained according to a similar manner to that of Preparation 8(1).

(2) 5-(N-Formyl-N-methoxycarbonylmethylamino)-1-methylpyrazole

NMR ($CDCl_3$, $\delta$) : 3.68 (3H, s), 3.77 (3H, s), 4.26 (2H, s), 6.16 (1H, d, J=3Hz), 7.39 (1H, d, J=3Hz), 8.15 (1H, s)

Preparation 9

(1) To a mixture of benzhydryl 7$\beta$-tert-butoxycarbonylamino-3-chloromethyl-3-cephem-4-carboxylate (20 g) and sodium iodide (5.82 g) in N,N-dimethylformamide (20 ml) was added 5-formamido-1-(2-formyloxyethyl)pyrazole (21.34 g) at ambient temperature. After being stirred for 24 hours at the same temperature, the mixture was poured into a mixture of water and ethyl acetate. The organic layer was separated and washed with water, aqueous sodium chloride solution, and dried over magnesium sulfate. The solution was evaporated in vacuo to give benzhydryl 7$\beta$-tert-butoxycarbonylamino-3-[3-formamido-2-(2-formyloxyethyl)-1-pyrazolio]methyl-3-cephem-4-carboxylate iodide (29.6 g).

IR (Nujol) : 1780, 1720 $cm^{-1}$

NMR (DMSO-$d_6$, $\delta$) : 1.49 (9H, s), 3.43 (2H, br s), 4.14-4.38 (2H, m), 4.52-4.73 (2H, m), 5.15 (1H, d, J=5Hz), 5.40 (2H, br s), 5.67 (1H, dd, J=5, 8Hz), 6.88 (1H, s), 7.02 (1H, d, J=3Hz), 7.18-7.52 (10H, m), 7.94 (1H, d, J=8Hz), 7.99 (1H, s), 8.27 (1H, d, J=3Hz), 8.51 (1H, br s)

The following compounds were obtained according to a similar manner to that of Preparation 9(1).

(2) Benzhydryl 7$\beta$-tert-butoxycarbonylamino-3-[3-formamido-2-(2-formyloxyethyl)-4-methyl-1-pyrazolio]-methyl-3-cephem-4-carboxylate iodide

IR (Nujol) : 3250, 1780, 1710, 1680 $cm^{-1}$

NMR (DMSO-$d_6$, $\delta$) : 1.53 (9H, s), 1.97 (3H, s), 3.51 (2H, br s), 4.04-4.42 (2H, m), 4.52-4.78 (2H, m), 5.08 (1H, d, J=5Hz), 5.39 (2H, br s), 5.61 (1H, dd, J=5, 8Hz), 6.86 (1H, s), 7.08-7.52 (10H, m), 7.93 (1H, s), 8.18 (1H, s), 8.34 (1H, s), 9.12 (1H, s)

(3) Benzhydryl 7$\beta$-tert-butoxycarbonylamino-3-[3-carbamoylamino-2-(2-formyloxyethyl)-1-pyrazolio]-methyl-3-cephem-4-carboxylate iodide

IR (Nujol) : 3300, 1780, 1710 $cm^{-1}$

NMR (DMSO-$d_6$, $\delta$) : 1.43 (9H, s), 3.5-3.8 (2H, m), 4.1-4.6 (4H, m), 5.14 (1H, d, J=5Hz), 5.33 (2H, s), 5.60 (1H, dd, J=8Hz, 5Hz), 6.66 (1H, s), 6.86 (1H, d, J=3Hz), 7.1-7.5 (10H, m), 7.93 (1H, d, J=8Hz), 8.00 (1H, s), 8.08 (1H, d, J=3Hz)

(4) Benzhydryl 7$\beta$-tert-butoxycarbonylamino-3-[3-acetamido-2-(2-acetoxyethyl)-1-pyrazolio]methyl-3-cephem-4-carboxylate iodide

IR (Nujol) : 1780, 1720, 1230 $cm^{-1}$

NMR (DMSO-$d_6$, $\delta$) : 1.41 (9H, s), 1.86 (3H, s), 2.25 (3H, s), 3.40 (2H, br s), 4-4.4 (4H, m), 5.12 (1H, d, J=5Hz), 5.37 (2H, s), 5.60 (1H, dd, J=8Hz, 5Hz), 6.85 (1H, s), 7.24 (1H, d, J=3Hz), 7.1-7.6 (10H, m), 7.90 (1H, d, J=8Hz), 8.21 (1H, d, J=3Hz), 11.17 (1H, s)

(5) Benzhydryl 7$\beta$-tert-butoxycarbonylamino-3-[2-(2-carbamoyloxyethyl)-3-formamido-1-pyrazolio)methyl-3-cephem-4-carboxylate iodide

(6) Benzhydryl 7-tert-butoxycarbonylamino-3-[3-(N-formyl-N-methylamino)-2-methyl-1-pyrazolio]methyl-3-cephem-4-carboxylate iodide

NMR (DMSO-$d_6$, $\delta$) : 1.38 (9H, s), 3.29 (3H, s), 3.68 (3H, s), 3.07-3.77 (2H, m), 5.18 (1H, d, J=5Hz), 5.35-5.75 (3H, m), 6.90 (1H, s), 7.01 (1H, d, J=2Hz), 7.08-7.60 (10H, m), 8.02 (1H, d, J=8Hz), 8.35 (1H, s), 8.43 (1H, d, J=2Hz)

(7) Benzhydryl 7-tert-butoxycarbonylamino-3-[3-(N-formyl-N-methoxycarbonylmethylamino)-2-methyl-1-pyrazolio]methyl-3-cephem-4-carboxylate iodide

IR (Nujol) : 3300, 1780, 1620 $cm^{-1}$

## Preparation 10

(1) To a solution of benzhydryl 7$\beta$-tert-butoxycarbonyl-amino-3-[3-formamido-2-(2-formyloxyethyl)-1-pyrazolio]methyl-3-cephem-4-carboxylate iodide (29.5 g) and anisole (30 ml) in methylene chloride (90 ml) was added dropwise trifluoroacetic acid (60 ml) under ice-cooling. After being stirred for 1 hour at ambient temperature, the mixture was poured into a mixture of diisopropyl ether (600 ml) and ethyl acetate (600 ml). The resultant precipitate was collected by filtration to give di(trifluoroacetic acid) salt of 7$\beta$-amino-3-[3-formamido-2-(2-formyloxyethyl)-1-pyrazolio]methyl-3-cephem-4-carboxylate (22.7 g).

IR (Nujol) : 1780, 1715, 1660 $cm^{-1}$

NMR (DMSO-$d_6$, $\delta$) : 3.53 (2H, br s), 4.28-4.56 (2H, m), 4.78-4.99 (2H, m), 5.29 (2H, br s), 5.53 (2H, br s), 7.14 (1H, d, J=3Hz), 8.22 (1H, s), 8.46 (1H, d, J=3Hz), 8.63 (1H, s)

The following compounds were obtained according to a similar manner to that of Preparation 10(1).

(2) Di(trifluoroacetic acid) salt of 7$\beta$-amino-3-[3-formamido-2-(2-formyloxyethyl)-4-methyl-1-pyrazolio]-methyl-3-cephem-4-carboxylate

IR (Nujol) : 1780, 1710, 1670 $cm^{-1}$

NMR (DMSO-$d_6$, $\delta$) : 1.98 (3H, s), 3.49 (2H, br s), 4.22-4.48 (2H, m), 4.61-4.87 (2H, m), 5.18 (2H, br s), 5.46 (2H, br s), 8.05 (1H, s), 8.23 (1H, s), 8.35 (1H, s)

(3) Di(trifluoroacetic acid) salt of 7$\beta$-amino-3-[3-carbamoylamino-2-(2-formyloxyethyl)-1-pyrazolio]methyl-3-cephem-4-carboxylate

IR (Nujol) : 3400, 1780, 1700, 1560 $cm^{-1}$

NMR (DMSO-$d_6$, $\delta$) : 3.46 (2H, br s), 4.2-4.6 (4H, m), 5.22 (2H, m), 5.40 (2H, s), 6.92 (1H, d, J=3Hz), 8.13 (1H, s), 8.20 (1H, d, J=3Hz)

(4) Di(trifluoroacetic acid) salt of 7$\beta$-amino-3-[3-acetamido-2-(2-acetoxyethyl)-1-pyrazolio]methyl-3-cephem-4-carboxylate

IR (Nujol) : 1780, 1660, 1190 $cm^{-1}$

NMR (DMSO-$d_6$, $\delta$) : 1.95 (3H, s), 2.23 (3H, s), 3.46 (2H, br s), 4.1-4.4 (4H, m), 5.20 (2H, m), 5.46 (2H, s), 7.01 (1H, d, J=3Hz), 8.27 (1H, d, J=3Hz), 11.17 (1H, s)

(5) Di(trifluoroacetic acid) salt of 7$\beta$-amino-3-[2-(2-carbamoyloxyethyl)-3-formamido-1-pyrazolio]methyl-3-cephem-4-carboxylate

(6) Di(trifluoroacetic acid) salt of 7$\beta$-amino-3-[3-(N-formyl-N-methylamino)-2-methyl-1-pyrazolio]methyl-3-cephem-4-carboxylate

NMR ($D_2O$, $\delta$) : 3.46 (3H, s), 3.87, 4.00 (total 3H, each s), 3.13-3.77 (2H, m), 5.05-5.47 (4H, m), 6.89 (1H, d, J=2Hz), 8.32 (1H, d, J=2Hz), 8.36 (1H, s)

(7) Di(trifluoroacetic acid) salt of 7$\beta$-amino-3-[3-(N-formyl-N-methoxycarbonylmethylamino)-2-methyl-1-pyrazolio]methyl-3-cephem-4-carboxylate

IR (Nujol) : 3300, 1780, 1610 $cm^{-1}$

## Preparation 11

(1) Concentrated hydrochloric acid (5.67 ml) was added to a mixture of di(trifluoroacetic acid) salt of 7$\beta$-amino-3-[3-formamido-2-(2-formyloxyethyl)-1-pyrazolio]methyl-3-cephem-4-carboxylate (10 g) in methanol (50 ml) at ambient temperature. After being stirred at the same temperature for 3 hours, the mixture was added dropwise to ethyl acetate (500 ml). The resultant precipitate was collected by filtration to give 7$\beta$-amino-3-[3-amino-2-(2-hydroxyethyl)-1-pyrazolio]methyl-3-cephem-4-carboxylate trihydrochloride (6.1 g).

IR (Nujol) : 3250, 1770, 1700, 1625 $cm^{-1}$

NMR (DMSO-$d_6$, $\delta$) : 3.43 (2H, br s), 3.52-3.88 (2H, m), 4.18-4.48 (2H, m), 5.28 (2H, br s), 5.37 (2H, br s), 5.97 (1H, d, J=3Hz), 8.18 (1H, d, J=3Hz)

The following compounds were obtained according to a similar manner to that of Preparation 11(1).

(2) 7$\beta$-Amino-3-[3-amino-2-(2-hydroxyethyl)-4-methyl-1-pyrazolio]methyl-3-cephem-4-carboxylate trihydrochloride

NMR (DMSO-$d_6$, $\delta$) : 1.94 (3H, s), 3.39 (2H, br s), 3.47-3.78 (2H, m), 4.06-4.42 (2H, m), 5.21 (4H, br s), 7.87 (1H, s)

(3) 7$\beta$-Amino-3-[3-carbamoylamino-2-(2-hydroxyethyl)-1-pyrazolio]methyl-3-cephem-4-carboxylate dihydrochloride

IR (Nujol) : 3300, 1770, 1700, 1560 $cm^{-1}$

NMR (DMSO-$d_6$, $\delta$) : 3.41 (2H, br s), 3.6-3.8 (4H, m), 5.20 (2H, m), 5.43 (2H, s), 6.85 (1H, d, J=3Hz), 8.20 (1H, d, J=3Hz)

(4) 7$\beta$-Amino-3-[3-amino-2-(2-carbamoyloxyethyl)-1-pyrazolio]methyl-3-cephem-4-carboxylate trihydrochloride

(5) 7$\beta$-Amino-3-[2-methyl-3-methylamino-1-pyrazolio]methyl-3-cephem-4-carboxylate trihydrochloride

NMR ($D_2O$-$NaHCO_3$, $\delta$) : 2.93 (3H, s), 3.25-3.38 (2H, m), 3.63 (3H, s), 5.07-5.33 (4H, m), 5.97 (1H, d, J=2Hz), 7.89 (1H, d, J=2Hz)

(6) 7$\beta$-Amino-3-[2-methyl-3-methoxycarbonylmethylamino-1-pyrazolio]methyl-3-cephem-4-carboxylate trihydrochloride

IR (Nujol) : 3200-3350, 1780, 1610 $cm^{-1}$

Example 1

To a solution of 7$\beta$-amino-3-[3-amino-2-(2-hydroxyethyl)-1-pyrazolio]methyl-3-cephem-4-carboxylate trihydrochloride (2 g) and N-(trimethylsilyl)acetamide (5.85 g) in tetrahydrofuran (40 ml) was added (Z)-2-(5-amino-1,2,4-thiadiazol-3-yl)-2-allyloxyiminoacetyl chloride hydrochloride (1.26 g) under ice-cooling. After being stirred for 1 hour at the same temperature, the reaction mixture was poured into diisopropyl ether (200 ml). The resulting precipitate was collected by filtration, dissolved in water (100 ml) and adjusted to pH 2.0 with 5% sodium bicarbonate solution. This solution was subjected to column chromatography on macroporous non-ionic adsorption resin "Diaion HP-20" (Trademark : prepared by Mitsubishi Chemical Industries). The desired compound was eluted with 10% isopropyl alcohol and lyophilized to give 7$\beta$-[2-(5-amino-1,2,4-thiadiazol-3-yl)-2-allyloxyiminoacetamido]-3-[3-amino-2-(2-hydroxyethyl)-1-pyrazolio]methyl-3-cephem-4-carboxylate (syn isomer) (1.07 g).

IR (Nujol) : 3150, 1760, 1660 $cm^{-1}$

NMR (DMSO-$d_6$, $\delta$) : 2.90 and 3.23 (2H, ABq, J=18Hz), 3.68 (2H, br s), 4.38 (2H, m), 4.63 (2H, d, J=5Hz), 5.02 (1H, d, J=5Hz), 5.09-5.47 (2H, m), 5.04 and 5.33 (2H, ABq, J=15Hz), 5.64 (1H, dd, J=5, 8Hz), 5.78-6.16 (1H, m), 5.81 (1H, d, J=3Hz), 7.26 (2H, br s), 8.08 (1H, d, J=3Hz), 8.10 (2H, br s), 9.50 (1H, d, J=8Hz)

Example 2

The following compounds were obtained according to a similar manner to that of Example 1.

(1) 7$\beta$-[2-(5-Amino-1,2,4-thiadiazol-3-yl)-2-methoxyiminoacetamido]-3-(2-methyl-3-amino-1-pyrazolio)-methyl-3-cephem-4-carboxylate (syn isomer)

IR (Nujol) : 3580, 3310, 3200, 1760, 1670, 1615, 1580 $cm^{-1}$

NMR (DMSO-$d_6$-$D_2O$, $\delta$) : 2.97, 3.23 (2H, ABq, J=18Hz), 3.68 (3H, s), 5.01 (1H, d, J=5Hz), 4.98, 5.28 (2H, ABq, J=15Hz), 5.66 (1H, d, J=5Hz), 5.85 (1H, d, J=3Hz), 8.01 (1H, d, J=3Hz)

(2) 7$\beta$-[2-(5-Amino-1,2,4-thiadiazol-3-yl)-2-methoxyiminoacetamido]-3-[2-(2-hydroxyethyl)-3-amino-1-pyrazolio]methyl-3-cephem-4-carboxylate (syn isomer)

IR (Nujol) : 3150-3300, 1760, 1580-1660 $cm^{-1}$

NMR (DMSO-$d_6$, $\delta$) : 2.92, 3.25 (2H, ABq, J=18Hz), 3.40-3.77 (3H, m), 3.80 (3H, s), 4.14-4.70 (2H, m), 4.90-5.42 (2H, m), 5.63 (1H, dd, J=5Hz, 8Hz), 5.83 (1H, d, J=3Hz), 7.17-7.57 (2H, br s), 8.08 (1H, d, J=3Hz), 7.93-8.30 (2H, br s), 9.49 (1H, d, J=8Hz)

(3) 7$\beta$-[2-(5-Amino-1,2,4-thiadiazol-3-yl)-2-allyloxyiminoacetamido]-3-[3-amino-2-(2-hydroxyethyl)-4-methyl-1-pyrazolio]methyl-3-cephem-4-carboxylate (syn isomer)

IR (Nujol) : 3250, 1760, 1640, 1600 $cm^{-1}$

NMR ($D_2O$, $\delta$) : 1.96 (3H, s), 3.03 and 3.33 (2H, ABq, J=18Hz), 3.69-3.33 (2H, m), 4.17-4.41 (2H, m), 4.78 (2H, d, J=5Hz), 5.03 (1H, d, J=5Hz), 5.04-5.48 (4H, m), 5.72-6.21 (1H, m), 5.81 (1H, d, J=5Hz), 7.68 (1H, s)

(4) 7$\beta$-[2-(5-Amino-1,2,4-thiadiazol-3-yl)-2-allyloxyiminoacetamido]-3-[3-carbamoylamino-2-(2-hydroxyethyl)-1-pyrazolio]methyl-3-cephem-4-carboxylate (syn isomer)

IR (Nujol) : 3300, 1770, 1700, 1560 $cm^{-1}$

NMR ($D_2O$, $\delta$) : 3.07 (1H, d, J=18Hz), 3.40 (1H, d, J=18Hz), 3.84 (4H, m), 4.74 (2H, d, J=5Hz), 5.20 (1H, d, J=5Hz), 5.25 (2H, s), 5.1-5.5 (2H, m), 5.79 (1H, d, J=5Hz), 5.9-6.4 (1H, m), 6.74 (1H, d, J=3Hz), 8.04 (1H, d, J=3Hz)

(5) 7$\beta$-[2-(5-Amino-1,2,4-thiadiazol-3-yl)-2-methoxyiminoacetamido]-3-[3-amino-2-(2-carbamoyloxyethyl)-1-pyrazolio]methyl-3-cephem-4-carboxylate (syn isomer)

IR (Nujol) : 3300, 1760, 1710, 1590, 1520 $cm^{-1}$

NMR ($D_2O$, $\delta$) : 3.07 (1H, d, J=18Hz), 3.42 (1H, d, J=18Hz), 4.06 (3H, s), 4.17-4.60 (4H, m), 4.70-5.43 (3H, m), 5.83 (1H, d, J=5Hz), 5.94 (1H, d, J=3Hz), 7.87 (1H, d, J=3Hz)

(6) 7$\beta$-[2-(5-Amino-1,2,4-thiadiazol-3-yl)-2-allyloxyiminoacetamido]-3-[3-amino-2-(2-carbamoyloxyethyl)-1-pyrazolio]methyl-3-cephem-4-carboxylate (syn isomer)

IR (Nujol) : 3300, 1770, 1710, 1600 $cm^{-1}$

NMR ($D_2O$, $\delta$) : 3.00-3.77 (2H, m), 4.27-4.67 (4H, m), 4.67-5.63 (7H, m), 5.96 (1H, d, J=5Hz), 6.06 (1H, d, J=3Hz), 7.99 (1H, d, J=3Hz)

(7) 7$\beta$-[2-(5-Amino-1,2,4-thiadiazol-3-yl)-2-allyloxyiminoacetamido]-3-[2-methyl-3-methylamino-1-pyrazolio]methyl-3-cephem-4-carboxylate (syn isomer)

IR (Nujol) : 3200-3300, 1770, 1670, 1610, 1520 $cm^{-1}$

NMR ($D_2O$, $\delta$) : 2.88 (3H, s), 3.58 (3H, s), 3.03-3.33 (2H, m), 4.50-4.87 (2H, m), 4.93-5.48 (3H, m), 5.70-5.97 (2H, m), 7.84 (1H, d, J=3Hz)

(8) 7$\beta$-[2-(5-Amino-1,2,4-thiadiazol-3-yl)-2-methoxyiminoacetamido]-3-(2-methyl-3-methylamino-1-pyrazolio)methyl-3-cephem-4-carboxylate (syn isomer)

IR (Nujol) : 3200-3300, 1760, 1660, 1610, 1520 $cm^{-1}$

NMR ($D_2O$, $\delta$) : 2.89 (3H, s), 3.58 (3H, s), 4.04 (3H, s), 3.03-3.30 (2H, m), 4.93-5.25 (2H, m), 5.77-5.97 (2H, m), 7.85 (1H, d, J=3Hz)

Example 3

To a solution of di(trifluoroacetic acid) salt of 7$\beta$-amino-3-[3-acetamido-2-(2-acetoxyethyl)-1-pyrazolio]-methyl-3-cephem-4-carboxylate (1 g) and N-(trimethylsilyl)acetamide (2.01 g) in dichloromethane (20 ml) was added (Z)-2-(5-amino-1,2,4-thiadiazol-3-yl)-2-allyloxyiminoacetyl chloride hydrochloride (0.520 g) under stirring and ice-cooling. The mixture was stirred for one hour at room temperature. The reaction mixture was added to ether under stirring and ice-cooling. The produced amorphous solid was dried in vacuo and dissolved in water. The aqueous solution was adjusted to pH 13 with 1N aqueous sodium hydroxide under stirring at -3 ~ 0 °C and stirred for 2 hours at the same temperature. The aqueous solution was adjusted to pH 2 with 1N hydrochloric acid and subjected to column chromatography on Diaion HP-20 and the elution was carried out with 10% aqueous isopropyl alcohol.
The fractions containing the object compound were combined, concentrated to remove isopropyl alcohol and lyophilized to give 7$\beta$-[2-(5-amino-1,2,4-thiadiazol-3-yl)-2-allyloxyiminoacetamido]-3-[3-acetamido-2-(2-hydroxyethyl)-1-pyrazolio]methyl-3-cephem-4-carboxylate (syn isomer) (222 mg).

mp : 150 °C (dec.)

IR (Nujol) : 3200, 1770, 1660, 1600 $cm^{-1}$

NMR ($D_2O$, $\delta$) : 2.22 (3H, s), 3.08 (1H,d, J=18Hz), 3.42 (1H, d, J=18Hz), 3.7-4.0 (4H, m), 4.74 (2H, d, J=6Hz), 5.20 (1H, d, J=5Hz), 5.30 (2H, s), 5.1-5.5 (2H, m), 5.81 (1H, d, J=5Hz), 5.8-6.4 (1H, m), 6.85 (1H, d, J=3Hz), 8.11 (1H, d, J=3Hz)

Example 4

To a solution of 7$\beta$-amino-3-[2-methyl-3-methoxycarbonylmethylamino-1-pyrazolio]methyl-3-cephem-4-carboxylate trihydrochloride (0.7 g) and N-(trimethylsilyl)acetamide (1.87 g) in tetrahydrofuran (14 ml) was added (Z)-2-(5-amino-1,2,4-thiadiazol-3-yl)-2-methoxyiminoacetyl chloride hydrochloride (0.37 g) under ice-cooling and the mixture was stirred at room temperature for 2 hours. The reaction mixture was poured into ethyl acetate (140 ml) and the resultant powder was collected by filtration. The powder was dissolved in ice-water and adjusted to pH 13 with 1N aqueous sodium hydroxide. After stirring for 20 minutes under ice-cooling, the solution was adjusted to pH 2 with 3N hydrochloric acid and subjected to column chromatography on "Diaion HP-20" using 5% aqueous isopropyl alcohol as an eluent. Fractions containing the object compound were combined, evaporated in vacuo to remove isopropyl alcohol and lyophilized to give 7$\beta$-[2-(5-amino-1,2,4-thiadiazol-3-yl)-2-methoxyiminoacetamido]-3-(3-carboxymethylamino-2-methyl-1-pyrazolio)-methyl-3-cephem-4-carboxylate (syn isomer) (0.24 g).

IR (Nujol) : 3250, 1760, 1670, 1610, 1520 $cm^{-1}$

NMR ($D_2O$, δ) : 3.10 and 3.38 (2H, ABq, J = 18Hz), 3.67 (3H, s), 3.83 (2H, s), 4.06 (3H, s), 4.97 and 5.28 (2H, ABq, J = 14Hz), 5.18 (1H, d, J = 5Hz), 5.82 (1H, d, J = 5Hz), 5.84 (1H, d, J = 3Hz), 7.85 (1H, d, J = 3Hz)

Example 5

The following compound was obtained according to a similar manner to that of Example 4.

7β-[2-(5-Amino-1,2,4-thiadiazol-3-yl)-2-allyloxyiminoacetamido]-3-(3-carboxymethylamino-2-methyl-1-pyrazolio)methyl-3-cephem-4-carboxylate (syn isomer)

IR (Nujol) : 3300, 1770, 1670, 1610, 1520 $cm^{-1}$

NMR ($D_2O$-$NaHCO_3$, δ) : 3.10 and 3.38 (2H, ABq, J = 18Hz), 3.67 (3H, s), 3.83 (2H, s), 4.67-4.93 (2H, m), 4.83-5.53 (2H, m), 5.19 (1H, d, J = 5Hz), 5.70-6.23 (3H, m), 7.85 (1H, d, J = 3Hz)

**Claims**

**Claims for the following Contracting States : AT, BE, CH, DE, FR, GB, IT, LI, LU, NL, SE**

**1.** A compound of the formula :

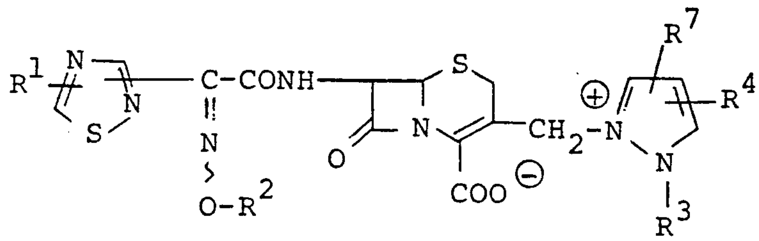

wherein

$R^1$ is amino or protected amino,
$R^2$ is $C_1$-$C_6$-alkyl or $C_1$-$C_6$-alkenyl,
$R^3$ is $C_1$-$C_6$-alkyl, hydroxy($C_1$-$C_6$)alkyl or protected hydroxy($C_1$-$C_6$)alkyl,
$R^4$ is amino, protected amino, $C_1$-$C_6$-alkylamino,protected $C_1$-$C_6$-alkylamino, carboxy($C_1$-$C_6$)-alkylamino, N-[protected carboxy($C_1$-$C_6$)alkyl]amino and
$R^7$ is hydrogen or $C_1$-$C_6$-alkyl, and

a pharmaceutically acceptable salt thereof.

**2.** A compound of claim 1,
wherein
$R^3$ is $C_1$-$C_6$-alkyl, hydroxy($C_1$-$C_6$)alkyl or acyloxy($C_1$-$C_6$)alkyl, and
$R^4$ is amino, acylamino, $C_1$-$C_6$-alkylamino, carboxy($C_1$-$C_6$)alkylamino or esterified carboxy($C_1$-$C_6$)alkylamino.

**3.** A compound of claim 2,
wherein
$R^3$ is $C_1$-$C_6$-alkyl, hydroxy($C_1$-$C_6$)alkyl, carbamoyloxy($C_1$-$C_6$)alkyl or $C_1$-$C_6$-alkanoyloxy($C_1$-$C_6$)-alkyl,
$R^4$ is amino, carbamoylamino, $C_1$-$C_6$-alkanoylamino, $C_1$-$C_6$-alkylamino, carboxy($C_1$-$C_6$)-alkylamino or $C_1$-$C_6$-alkoxycarbonyl($C_1$-$C_6$)alkylamino.

**4.** A compound of claim 3,
wherein
$R^1$ is amino,
$R^2$ is $C_1$-$C_6$-alkyl or $C_1$-$C_6$-alkenyl,
$R^3$ is $C_1$-$C_6$-alkyl, hydroxy($C_1$-$C_6$)alkyl or carbamoyloxy($C_1$-$C_6$)alkyl,
$R^4$ is amino, carbamoylamino, $C_1$-$C_6$-alkanoylamino, $C_1$-$C_6$-alkylamino or carboxy($C_1$-$C_6$)-alkylamino, and

$R^7$ is hydrogen or $C_1$-$C_6$-alkyl.

**5.** A compound of claim 4, which is 7β-[2-(5-Amino-1,2,4-thiadiazol-3-yl)-2-methoxyimino-acetamido]-3-(3-amino-2-methyl-1-pyrazolio)-methyl-3-cephem-4-carboxylate (syn isomer).

**6.** A process for preparing a compound of the formula :

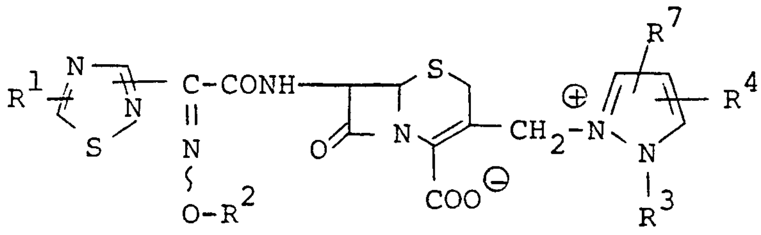

wherein
- $R^1$ is amino or protected amino,
- $R^2$ is $C_1$-$C_6$-alkyl or $C_1$-$C_6$-alkenyl,
- $R^3$ is $C_1$-$C_6$-alkyl, hydroxy($C_1$-$C_6$)alkyl or protected hydroxy($C_1$-$C_6$)alkyl,
- $R^4$ is amino, protected amino, $C_1$-$C_6$-alkylamino,protected $C_1$-$C_6$-alkylamino, carboxy($C_1$-$C_6$)-alkylamino, N-[protected carboxy($C_1$-$C_6$)alkyl]amino and
- $R^7$ is hydrogen or $C_1$-$C_6$-alkyl,

or a salt thereof,
which comprises
(1) reacting a compound of the formula :

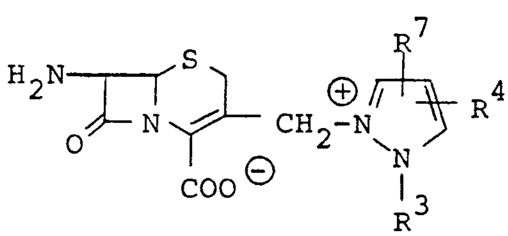

wherein $R^3$, $R^4$ and $R^7$ are each as defined above,
or its reactive derivative at the amino group or a salt thereof with a compound of the formula :

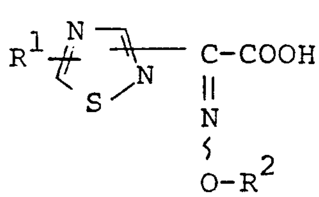

wherein $R^1$ and $R^2$ are each as defined above,
or its reactive derivative at the carboxy group or a salt thereof to give a compound of the formula :

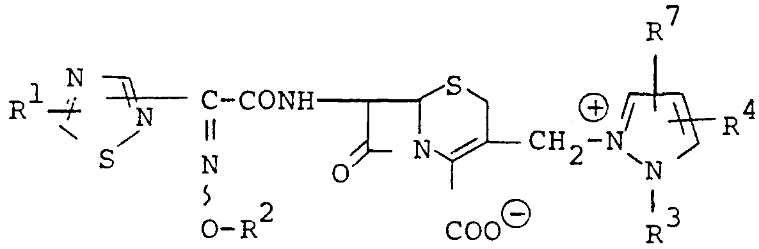

wherein

$R^1$, $R^2$, $R^3$, $R^4$ and $R^7$ are each as defined above,

or a salt thereof, or

(2) subjecting a compound of the formula :

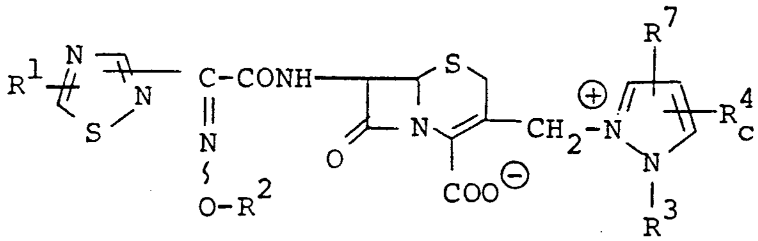

wherein

$R^1$, $R^2$, $R^3$ and $R^7$ are each as defined above,

$R^4_c$ is N-[protected carboxy($C_1$-$C_6$)alkyl]amino,

or a salt thereof to elimination reaction of the carboxy protective group to give a compound of the formula :

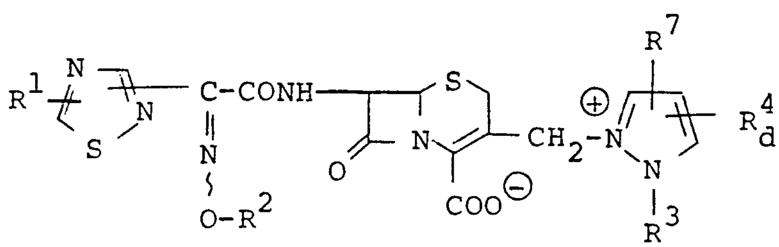

wherein

$R^1$, $R^2$, $R^3$ and $R^7$ are each defined above,

$R^4_d$ is carboxy($C_1$-$C_6$)alkylamino,

or a salt thereof, or

(3) subjecting a compound of the formula :

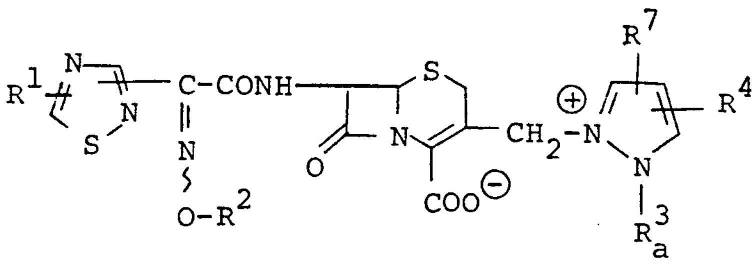

wherein

$R^1$, $R^2$, $R^4$ and $R^7$ are each as defined above,

$R^3_a$ is protected hydroxy($C_1$-$C_6$)alkyl,

or a salt thereof to elimination reaction of the hydroxy protective group to give a compound of the formula :

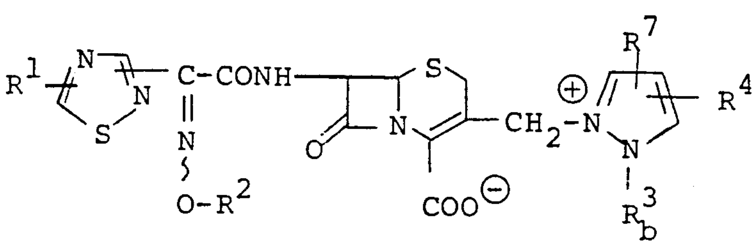

wherein

$R^1$, $R^2$, $R^4$ and $R^7$ are each as defined above,

$R^3_b$ is hydroxy($C_1$-$C_6$)alkyl,

or a salt thereof.

**7.** A pharmaceutical composition which comprises, as an active ingredient, a compound of claim 1 or a pharmaceutically acceptable salt thereof in admixture with pharmaceutically acceptable carriers.

**8.** A compound of claim 1 or a pharmaceutically acceptable salt thereof for use as a medicament.

**9.** A compound of claim 1 or a pharmceutically acceptable salt thereof for use as an antimicrobial agent.

**10.** A use of a compound of claim 1 or a pharmaceutically acceptable salt thereof for manufacture of medicament for treating infectious diseases.

**Claim for the following Contracting State : ES**

**1.** A process for preparing a compound of the formula :

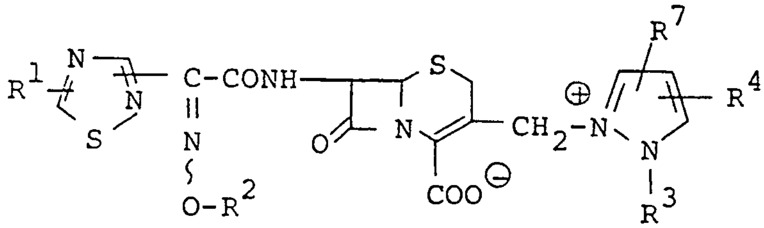

wherein

$R^1$ is amino or protected amino,

$R^2$ is $C_1$-$C_6$-alkyl or $C_1$-$C_6$-alkenyl,

$R^3$ is $C_1$-$C_6$-alkyl, hydroxy($C_1$-$C_6$)alkyl or protected hydroxy($C_1$-$C_6$)alkyl,

$R^4$ is amino, protected amino, $C_1$-$C_6$-alkylamino,protected $C_1$-$C_6$-alkylamino, carboxy($C_1$-$C_6$)-alkylamino, N-[protected carboxy($C_1$-$C_6$)alkyl]amino and

$R^7$ is hydrogen or $C_1$-$C_6$-alkyl,

or a salt thereof,

which comprises

(1) reacting a compound of the formula :

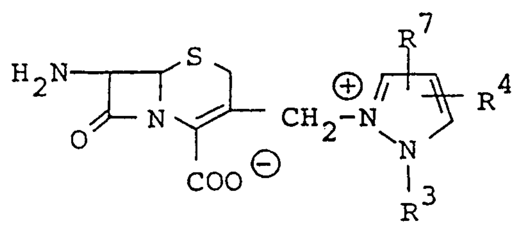

wherein $R^3$, $R^4$ and $R^7$ are each as defined above,

or its reactive derivative at the amino group or a salt thereof with a compound of the formula :

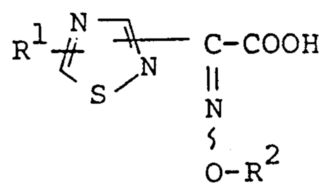

wherein $R^1$ and $R^2$ are each as defined above,

or its reactive derivative at the carboxy group or a salt thereof to give a compound of the formula :

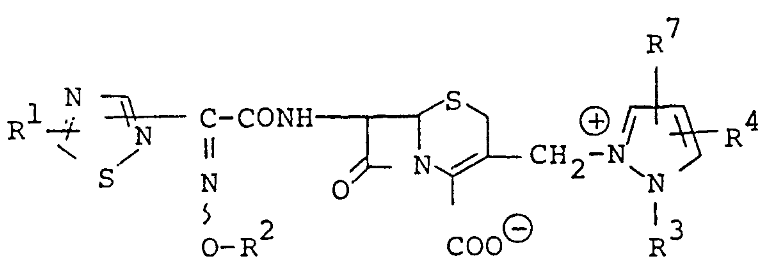

wherein

$R^1$, $R^2$, $R^3$, $R^4$ and $R^7$ are each as defined above,

or a salt thereof, or

(2) subjecting a compound of the formula :

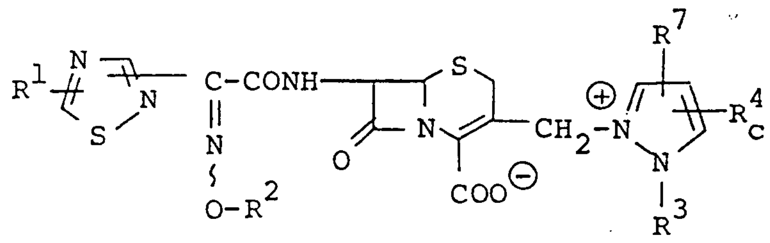

wherein

$R^1$, $R^2$, $R^3$ and $R^7$ are each as defined above,

$R^4_c$ is N-[protected carboxy($C_1$-$C_6$)alkyl]amino,

or a salt thereof to elimination reaction of the carboxy protective group to give a compound of the formula :

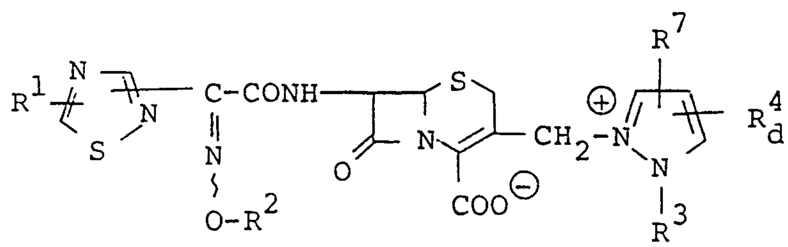

wherein

$R^1$, $R^2$, $R^3$ and $R^7$ are each as defined above,

$R^4_d$ is carboxy($C_1$-$C_6$)alkylamino,

or a salt thereof, or

(3) subjecting a compound of the formula :

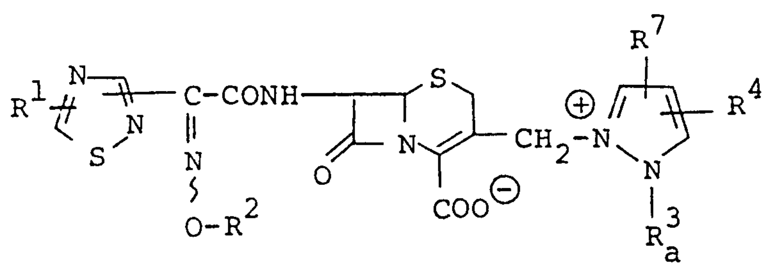

wherein

$R^1$, $R^2$, $R^4$ and $R^7$ are each as defined above,

$R^3_a$ is protected hydroxy($C_1$-$C_6$)alkyl,

or a salt thereof to elimination reaction of the hydroxy protective group to give a compound of the formula :

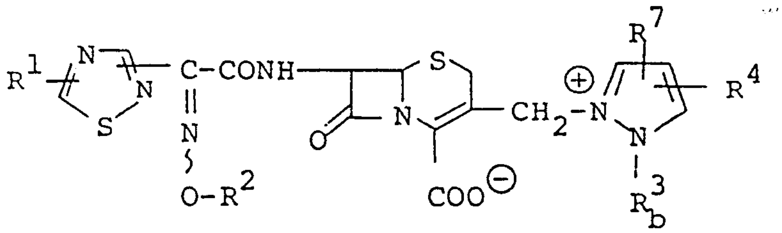

wherein
$R^1$, $R^2$, $R^4$ and $R^7$ are each as defined above,
$R^3_b$ is hydroxy($C_1$-$C_6$)alkyl,
or a salt thereof.

**Claims for the following Contracting State : GR**

**1.** A process for preparing a compound of the formula :

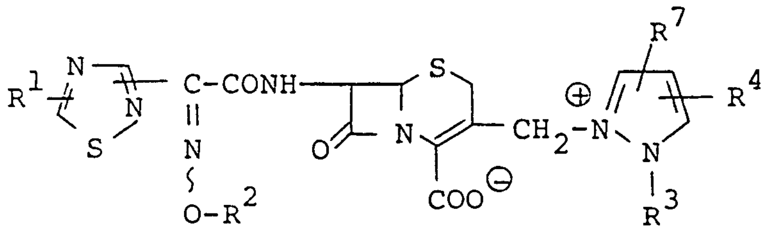

wherein
$R^1$ is amino or protected amino,
$R^2$ is $C_1$-$C_6$-alkyl or $C_1$-$C_6$-alkenyl,
$R^3$ is $C_1$-$C_6$-alkyl, hydroxy($C_1$-$C_6$)alkyl or protected hydroxy($C_1$-$C_6$)alkyl,
$R^4$ is amino, protected amino, $C_1$-$C_6$-alkylamino,protected $C_1$-$C_6$-alkylamino, carboxy($C_1$-$C_6$)-alkylamino, N-[protected carboxy($C_1$-$C_6$)alkyl]amino and
$R^7$ is hydrogen or $C_1$-$C_6$-alkyl,
or a salt thereof,
which comprises
(1) reacting a compound of the formula :

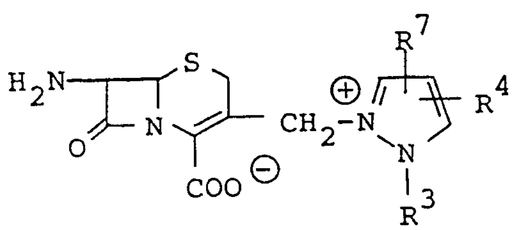

wherein $R^3$, $R^4$ and $R^7$ are each as defined above,
or its reactive derivative at the amino group or a salt thereof with a compound of the formula :

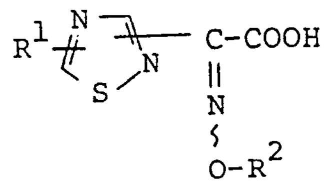

wherein $R^1$ and $R^2$ are each as defined above,
or its reactive derivative at the carboxy group or a salt thereof to give a compound of the formula :

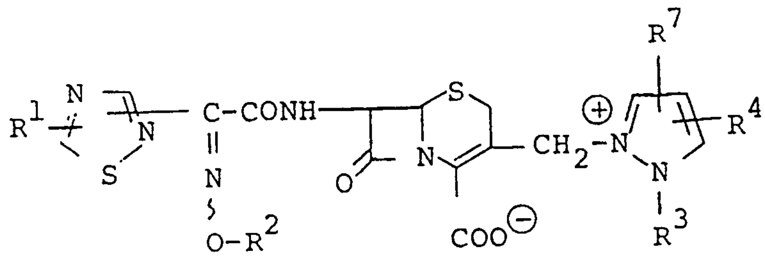

wherein
$R^1$, $R^2$, $R^3$, $R^4$ and $R^7$ are each as defined above,
or a salt thereof, or
(2) subjecting a compound of the formula :

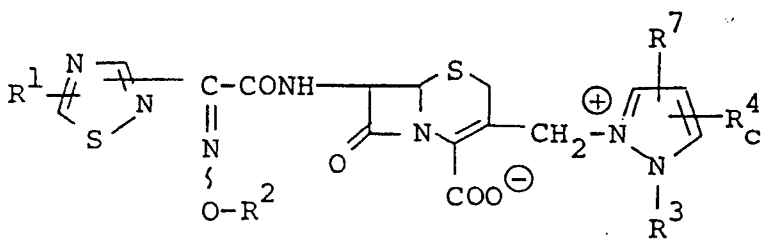

wherein
$R^1$, $R^2$, $R^3$ and $R^7$ are each as defined above,
$R^4_c$ is N-[protected carboxy($C_1$-$C_6$)alkyl]amino,
or a salt thereof to elimination reaction of the carboxy protective group to give a compound of the formula :

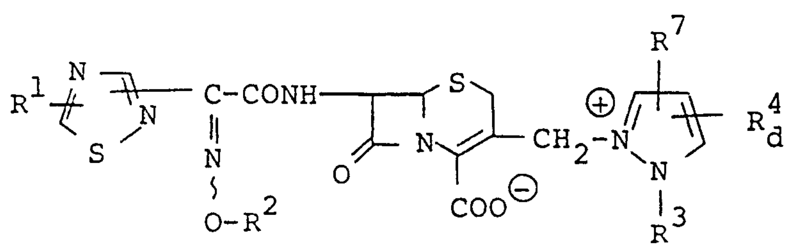

wherein
$R^1$, $R^2$, $R^3$ and $R^7$ are each as defined above,
$R^4_d$ is carboxy($C_1$-$C_6$)alkylamino,

or a salt thereof, or

(3) subjecting a compound of the formula :

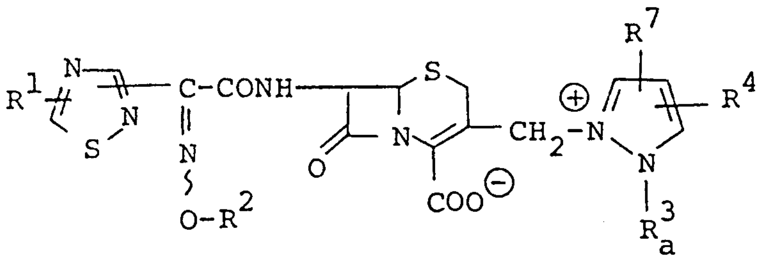

wherein

$R^1$, $R^2$, $R^4$ and $R^7$ are each as defined above,

$R^3_a$ is protected hydroxy($C_1$-$C_6$)alkyl,

or a salt thereof to elimination reaction of the hydroxy protective group to give a compound of the formula :

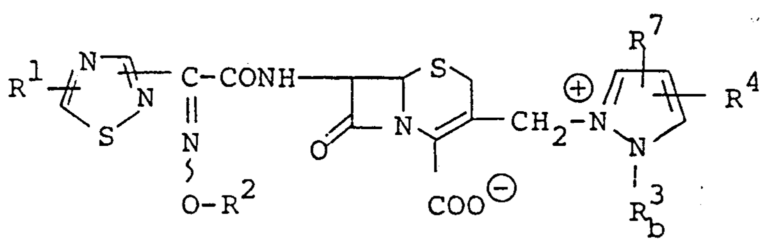

wherein

$R^1$, $R^2$, $R^4$ and $R^7$ are each as defined above,

$R^3_b$ is hydroxy($C_1$-$C_6$)alkyl,

or a salt thereof.

**2.** Modification of the process of claim 1 which is characterized by bringing a compound prepared by the process of claim 1 into a pharmaceutically acceptable form by admixture or presentation of said compound with pharmaceutically acceptable carriers.

**Patentansprüche**

**Patentansprüche für folgende Vertragsstaaten : AT, BE, CH, DE, FR, GB, LI, IT, LU, NL, SE**

**1.** Verbindung der Formel:

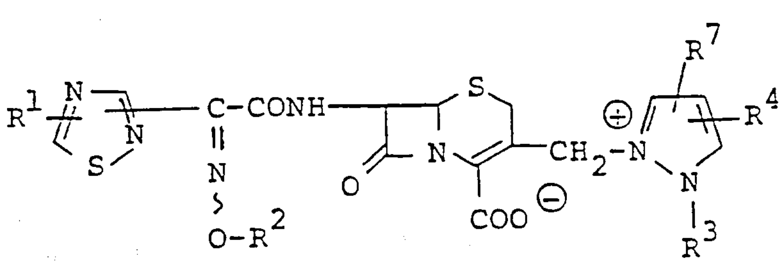

worin $R^1$ Amino oder geschütztes Amino ist, $R^2$ ($C_1$-$C_6$)Alkyl oder ($C_1$-$C_6$)Alkenyl ist, $R^3$ ($C_1$-$C_6$)Alkyl, Hydroxy($C_1$-$C_6$)alkyl oder geschütztes Hydroxy($C_1$-$C_6$)alkyl ist, $R^4$ Amino, geschütztes Amino, ($C_1$-$C_6$)-Alkylamino, geschütztes ($C_1$-$C_6$)Alkylamino, Carboxy($C_1$-$C_6$)alkylamino, N-[geschütztes Carboxy($C_1$-$C_6$)alkyl]amino ist und $R^7$ Wasserstoff oder ($C_1$-$C_6$)Alkyl ist, und ein pharmazeutisch verträgliches Salz davon.

2. Verbindung nach Anspruch 1, worin $R^3$ $(C_1-C_6)$Alkyl, Hydroxy$(C_1-C_6)$alkyl oder Acyloxy$(C_1-C_6)$alkyl ist und $R^4$ Amino, Acylamino, $(C_1-C_6)$Alkylamino, Carboxy$(C_1-C_6)$alkylamino oder verestertes Carboxy$(C_1-C_6)$alkylamino ist.

3. Verbindung nach Anspruch 2, worin $R^3$ $(C_1-C_6)$Alkyl, Hydroxy$(C_1-C_6)$alkyl, Carbamoyloxy$(C_1-C_6)$alkyl oder $(C_1-C_6)$Alkanoyloxy$(C_1-C_6)$alkyl ist, $R^4$ Amino, Carbamoylamino, $(C_1-C_6)$Alkanoylamino, $(C_1-C_6)$-Alkylamino, Carboxy$(C_1-C_6)$alkylamino oder $(C_1-C_6)$Alkoxycarbonyl$(C_1-C_6)$alkylamino ist.

4. Verbindung nach Anspruch 3, worin $R^1$ Amino ist, $R^2$ $(C_1-C_6)$Alkyl oder $(C_1-C_6)$Alkenyl ist, $R^3$ $(C_1-C_6)$-Alkyl, Hydroxy$(C_1-C_6)$alkyl oder Carbamoyloxy$(C_1-C_6)$alkyl ist, $R^4$ Amino, Carbamoylamino, $(C_1-C_6)$-Alkanoylamino, $(C_1-C_6)$Alkylamino oder Carboxy$(C_1-C_6)$alkylamino ist und $R^7$ Wasserstoff oder $(C_1-C_6)$-Alkyl ist.

5. Verbindung nach Anspruch 4, die 7β-[2-(5-Amino-1,2,4-thiadiazol-3-yl)-2-methoxyiminoacetamido]-3-(3-amino-2-methyl-1-pyrazolio)methyl-3-cephem-4-carboxylat (Syn-Isomer) ist.

6. Verfahren zur Herstellung einer Verbindung der Formel:

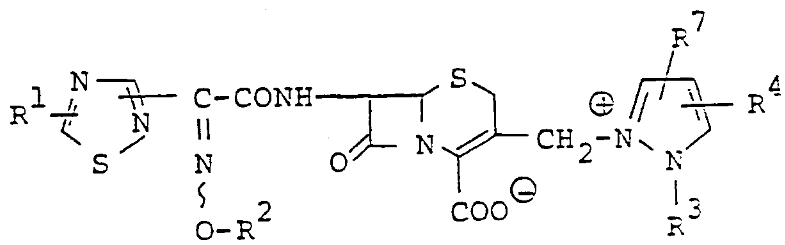

worin $R^1$ Amino oder geschütztes Amino ist, $R^2$ $(C_1-C_6)$Alkyl oder $(C_1-C_6)$Alkenyl ist, $R^3$ $(C_1-C_6)$Alkyl, Hydroxy$(C_1-C_6)$alkyl oder geschütztes Hydroxy$(C_1-C_6)$alkyl ist, $R^4$ Amino, geschütztes Amino, $(C_1-C_6)$-Alkylamino, geschütztes $(C_1-C_6)$Alkylamino, Carboxy$(C_1-C_6)$alkylamino, N-[geschütztes Carboxy$(C_1-C_6)$alkyl]amino ist und $R^7$ Wasserstoff oder $(C_1-C_6)$Alkyl ist oder ein Salz davon, welches umfaßt:

(1) Reagieren einer Verbindung der Formel:

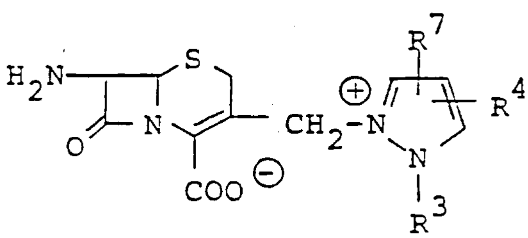

worin $R^3$, $R^4$ und $R^7$ jeweils wie oben definiert sind, oder ihres reaktiven Derivates an der Aminogruppe oder eines Salzes davon mit einer Verbindung der Formel:

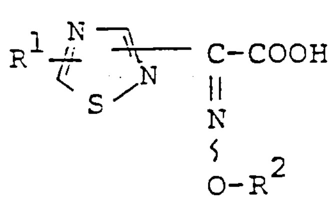

worin $R^1$ und $R^2$ jeweils wie oben definiert sind, oder ihrem reaktiven Derivat an der Carboxygruppe oder einem Salz davon, um eine Verbindung der Formel:

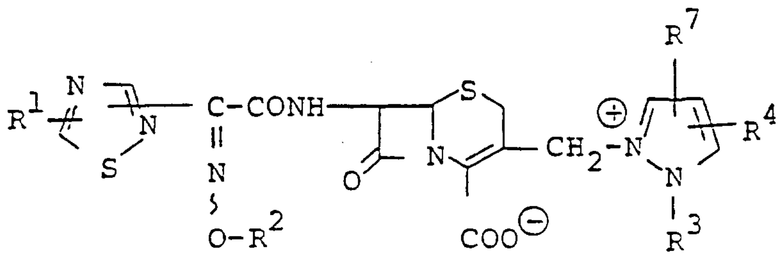

worin $R^1$, $R^2$, $R^3$, $R^4$ und $R^7$ jeweils wie oben definiert sind, oder ein Salz davon zu erhalten oder
(2) Unterwerfen einer Verbindung der Formel:

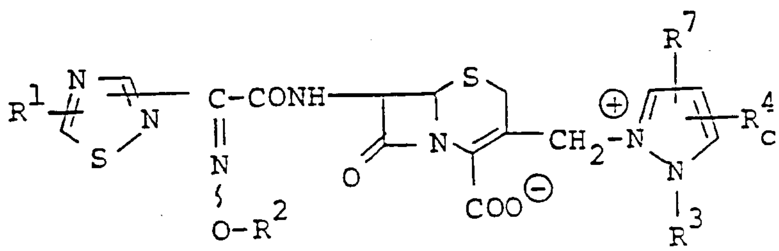

worin $R^1$, $R^2$, $R^3$ und $R^7$ jeweils wie oben definiert sind, $R^4{}_c$ N-[geschütztes Carboxy($C_1$-$C_6$)alkyl]-amino ist, oder eines Salzes davon der Eliminierungsreaktion der Carboxyschutzgruppe, um eine Verbindung der Formel:

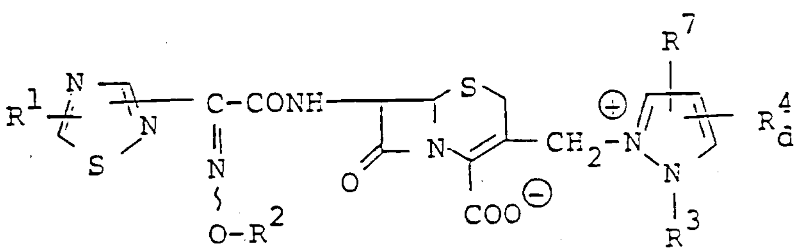

worin $R^1$, $R^2$, $R^3$ und $R^7$ jeweils wie oben definiert sind, $R^4{}_d$ Carboxy($C_1$-$C_6$)alkylamino ist, oder ein Salz davon zu erhalten oder
(3) Unterwerfen einer Verbindung der Formel:

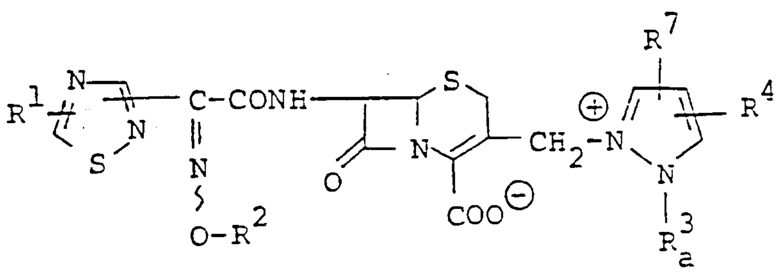

worin $R^1$, $R^2$, $R^4$ und $R^7$ jeweils wie oben definiert sind, $R^3{}_a$ geschütztes Hydroxy($C_1$-$C_6$)alkyl ist, oder eines Salzes davon der Eliminierungsreaktion der Hydroxyschutzgruppe, um eine Verbindung der Formel:

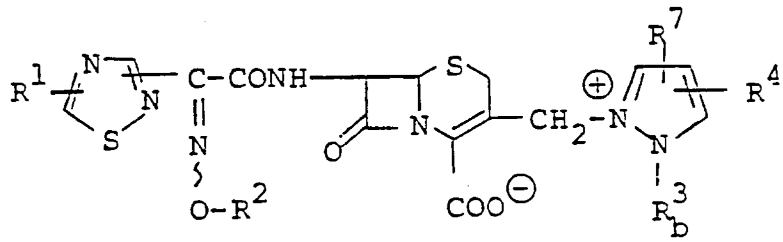

worin $R^1$, $R^2$, $R^4$ und $R^7$ jeweils wie oben definiert sind, $R^3{}_b$ Hydroxy($C_1$-$C_6$)alkyl ist oder ein Salz davon zu erhalten.

7. Pharmazeutische Zusammensetzung, die als aktiven Bestandteil eine Verbindung nach Anspruch 1 oder ein pharmazeutisch verträgliches Salz davon in Zusammenmischung mit pharmazeutisch verträglichen Trägern umfaßt.

8. Verbindung nach Anspruch 1 oder ein pharmazeutisch verträgliches Salz davon zur Verwendung als Medikament.

9. Verbindung nach Anspruch 1 oder ein pharmazeutisch verträgliches Salz davon zur Verwendung als antimikrobielles Mittel.

10. Verwendung einer Verbindung nach Anspruch 1 oder eines pharmazeutisch verträglichen Salzes davon zur Herstellung eines Medikaments zur Behandlung infektiöser Krankheiten.

**Patentanspruch für folgenden Vertragsstaat : ES**

1. Verfahren zur Herstellung einer Verbindung der Formel:

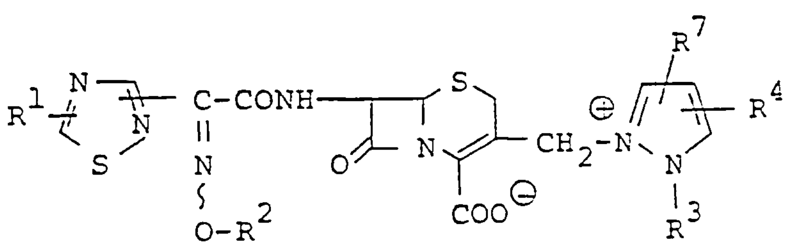

worin $R^1$ Amino oder geschütztes Amino ist, $R^2$ ($C_1$-$C_6$)Alkyl oder ($C_1$-$C_6$)Alkenyl ist, $R^3$ ($C_1$-$C_6$)Alkyl, Hydroxy($C_1$-$C_6$)alkyl oder geschütztes Hydroxy($C_1$-$C_6$)alkyl ist, $R^4$ Amino, geschütztes Amino, ($C_1$-$C_6$)-Alkylamino, geschütztes ($C_1$-$C_6$)Alkylamino, Carboxy($C_1$-$C_6$)alkylamino, N-[geschütztes Carboxy($C_1$-$C_6$)alkyl]amino ist und $R^7$ Wasserstoff oder ($C_1$-$C_6$)Alkyl ist oder ein Salz davon, welches umfaßt:

(1) Reagieren einer Verbindung der Formel:

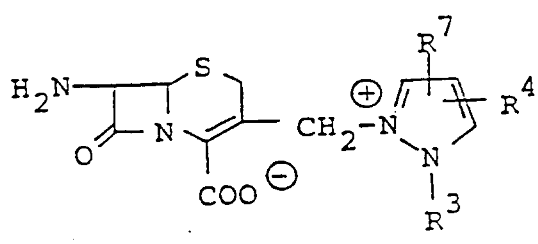

worin $R^3$, $R^4$ und $R^7$ jeweils wie oben definiert sind, oder ihres reaktiven Derivates an der Aminogruppe oder eines Salzes davon mit einer Verbindung der Formel:

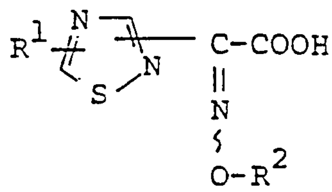

worin $R^1$ und $R^2$ jeweils wie oben definiert sind, oder ihrem reaktiven Derivat an der Carboxygruppe oder einem Salz davon, um eine Verbindung der Formel:

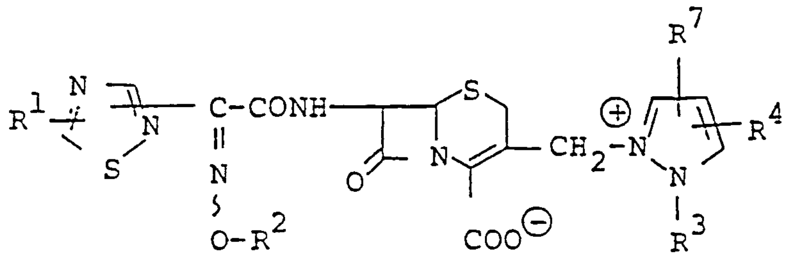

worin $R^1$, $R^2$, $R^3$, $R^4$ und $R^7$ jeweils wie oben definiert sind, oder ein Salz davon zu erhalten oder

(2) Unterwerfen einer Verbindung der Formel:

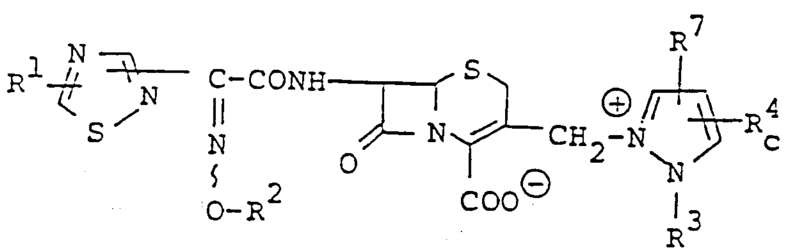

worin $R^1$, $R^2$, $R^3$ und $R^7$ jeweils wie oben definiert sind, $R^4{}_c$ N-[geschütztes Carboxy($C_1$-$C_6$)alkyl]-amino ist, oder eines Salzes davon der Eliminierungsreaktion der Carboxyschutzgruppe, um eine Verbindung der Formel:

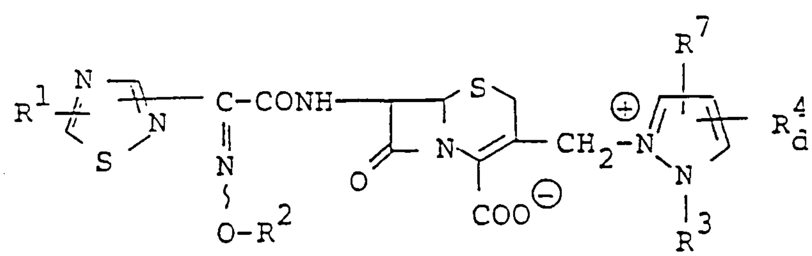

worin $R^1$, $R^2$, $R^3$ und $R^7$ jeweils wie oben definiert sind, $R^4{}_d$ Carboxy($C_1$-$C_6$)alkylamino ist, oder ein Salz davon zu erhalten oder

(3) Unterwerfen einer Verbindung der Formel:

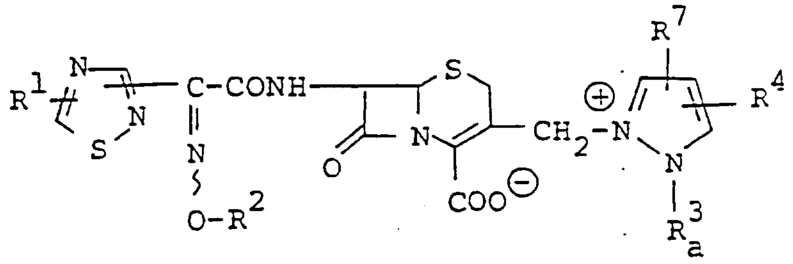

worin $R^1$, $R^2$, $R^4$ und $R^7$ jeweils wie oben definiert sind, $R^3_a$ geschütztes Hydroxy($C_1$-$C_6$)alkyl ist, oder eines Salzes davon der Eliminierungsreaktion der Hydroxyschutzgruppe, um eine Verbindung der Formel:

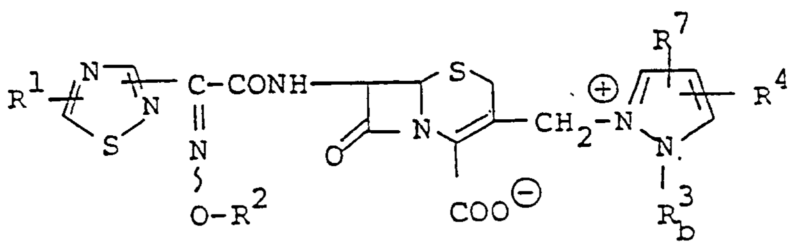

worin $R^1$, $R^2$, $R^4$ und $R^7$ jeweils wie oben definiert sind, $R^3_b$ Hydroxy($C_1$-$C_6$)alkyl ist, oder ein Salz davon zu erhalten.

**Patentansprüche für folgenden Vertragsstaat : GR**

**1.** Verfahren zur Herstellung einer Verbindung der Formel:

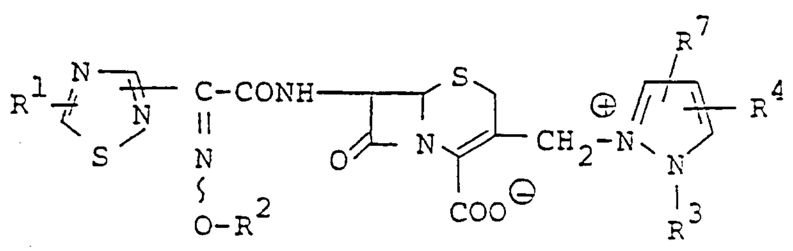

worin $R^1$ Amino oder geschütztes Amino ist, $R^2$ ($C_1$-$C_6$)Alkyl oder ($C_1$-$C_6$)Alkenyl ist, $R^3$ ($C_1$-$C_6$)Alkyl, Hydroxy($C_1$-$C_6$)alkyl oder geschütztes Hydroxy($C_1$-$C_6$)alkyl ist, $R^4$ Amino, geschütztes Amino, ($C_1$-$C_6$)-Alkylamino, geschütztes ($C_1$-$C_6$)Alkylamino, Carboxy($C_1$-$C_6$)alkylamino, N-[geschütztes Carboxy($C_1$-$C_6$)alkyl]amino ist und $R^7$ Wasserstoff oder ($C_1$-$C_6$)Alkyl ist, oder eines Salzes davon, welches umfaßt:

(1) Reagieren einer Verbindung der Formel:

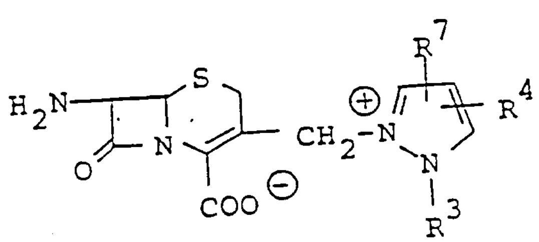

worin $R^3$, $R^4$ und $R^7$ jeweils wie oben definiert sind, oder ihres reaktiven Derivates an der Aminogruppe oder eines Salzes davon mit einer Verbindung der Formel:

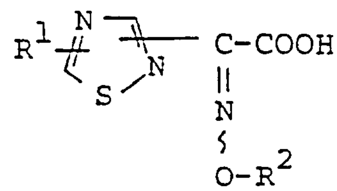

worin $R^1$ und $R^2$ jeweils wie oben definiert sind, oder ihrem reaktiven Derivat an der Carboxygruppe oder einem Salz davon, um eine Verbindung der Formel:

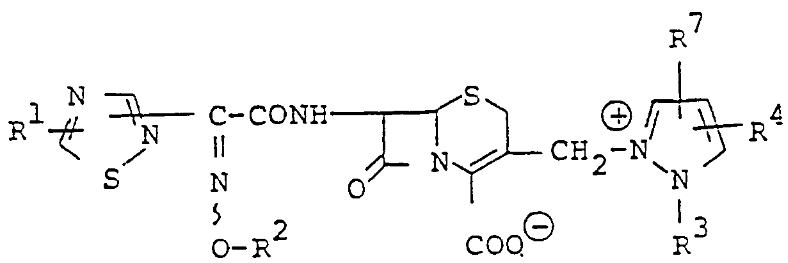

worin $R^1$, $R^2$, $R^3$, $R^4$ und $R^7$ jeweils wie oben definiert sind, oder ein Salz davon zu erhalten oder
(2) Unterwerfen einer Verbindung der Formel:

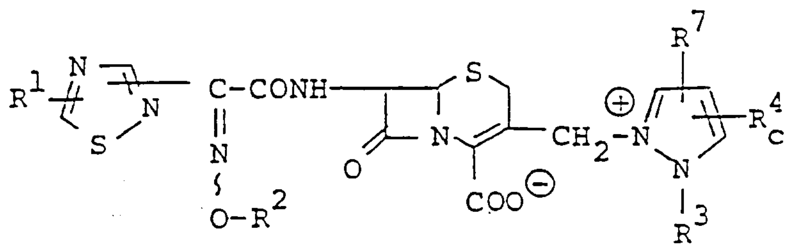

worin $R^1$, $R^2$, $R^3$ und $R^7$ jeweils wie oben definiert sind, $R^4{}_c$ N-[geschütztes Carboxy($C_1$-$C_6$)alkyl]-amino ist, oder eines Salzes davon der Eliminierungsreaktion der Carboxyschutzgruppe, um eine Verbindung der Formel:

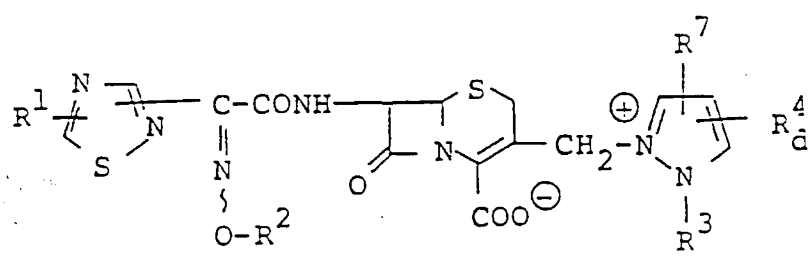

worin $R^1$, $R^2$, $R^3$ und $R^7$ jeweils wie oben definiert sind, $R^4{}_d$ Carboxy($C_1$-$C_6$)alkylamino ist, oder ein Salz davon zu erhalten oder

(3) Unterwerfen einer Verbindung der Formel:

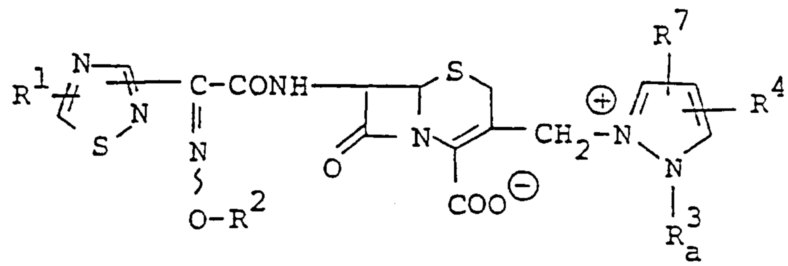

worin $R^1$, $R^2$, $R^4$ und $R^7$ jeweils wie oben definiert sind, $R^3_a$ geschütztes Hydroxy($C_1$-$C_6$)alkyl ist, oder eines Salzes davon der Eliminierungsreaktion der Hydroxyschutzgruppe, um eine Verbindung der Formel:

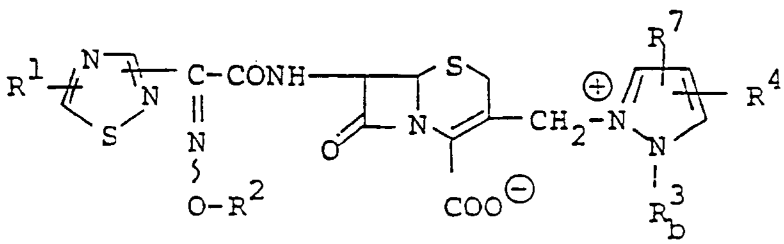

worin $R^1$, $R^2$, $R^4$ und $R^7$ jeweils wie oben definiert sind, $R^3_b$ Hydroxy($C_1$-$C_6$)alkyl ist, oder ein Salz davon zu erhalten.

**2.** Modifizierung des Verfahrens nach Anspruch 1, dadurch charakterisiert, daß eine Verbindung, hergestellt durch das Verfahren nach Anspruch 1, durch Zusammenmischung oder Präsentation dieser Verbindung mit pharmazeutisch verträglichen Trägern in eine passende pharmazeutische Form gebracht wird.

**Revendications**
**Revendications pour les Etats contractants suivants : AT, BE, CH, DE, FR, GB, LI, IT, LU, NL, SE**

**1.** Composé de la formule :

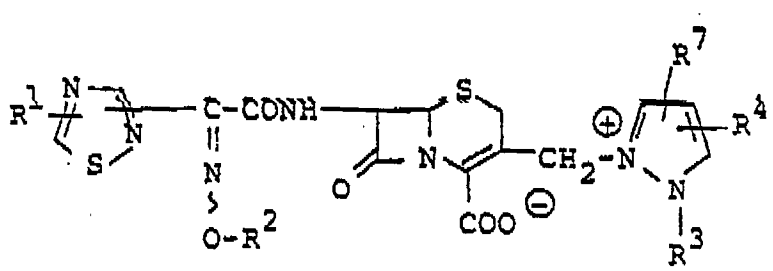

dans laquelle
$R^1$ est un groupe amino ou amino protégé,
$R^2$ est un groupe alkyle en $C_1$ à $C_6$ ou alcényle en $C_1$ à $C_6$,
$R^3$ est un groupe alkyle en $C_1$ à $C_6$, alkyl (en $C_1$ à $C_6$)hydroxy, ou alkyl(en $C_1$ à $C_6$)hydroxy protégé,
$R^4$ est un groupe amino, amino protégé, alkyl(en $C_1$ à $C_6$)amino, alkyl(en $C_1$ à $C_6$)amino protégé, alkyl(en $C_1$ à $C_6$)amino carboxy, N-[alkyl(en $C_1$ à $C_6$)carboxy protégé]amino et
$R^7$ est un atome d'hydrogène ou un groupe alkyle en $C_1$ à $C_6$, et
un de ses sels pharmaceutiquement acceptable.

**2.** Composé de la revendication 1,
dans lequel
$R^3$ est un groupe alkyle en $C_1$ à $C_6$, alkyl(en $C_1$ à $C_6$)hydroxy ou alkyl(en $C_1$ à $C_6$)acyloxy, et
$R^4$ est un groupe amino, acylamino, alkyl(en $C_1$ à $C_6$)amino, alkyl(en $C_1$ à $C_6$)amino carboxy, ou alkyl(en $C_1$ à $C_6$)amino carboxy estérifié.

**3.** Composé de la revendication 2,
dans lequel
$R^3$ est un groupe alkyle en $C_1$ à $C_6$, alkyl(en $C_1$ à $C_6$)hydroxy, alkyl(en $C_1$ à $C_6$)carbamoyloxy ou alkyl(en $C_1$ à $C_6$)alcanoyloxy,
$R^4$ est un groupe amino, carbamoylamino, alcanoyl(en $C_1$ à $C_6$)amino, alkyl(en $C_1$ à $C_6$)amino, alkyl(en $C_1$ à $C_6$)amino carboxy, ou alkyl(en $C_1$ à $C_6$)amino alcoxy (en $C_1$ à $C_6$)carbonyle.

**4.** Composé de la revendication 3 :
dans lequel
$R^1$ est un groupe amino;
$R^2$ est un groupe alkyle en $C_1$ à $C_6$ ou alcényle en $C_1$ à $C_6$,
$R^3$ est un groupe alkyle en $C_1$ à $C_6$, alkyl (en $C_1$ à $C_6$)hydroxy, ou alkyl (en $C_1$ à $C_6$)-carbamoyloxy,
$R^4$ est un groupe amino, carbamoylamino, alcanoyl(en $C_1$ à $C_6$)amino, alkyl(en $C_1$ à $C_6$)amino, ou alkyl(en $C_1$ à $C_6$)amino carboxy, et,
$R^7$ est un atome d'hydrogène ou un groupe alkyle en $C_1$ à $C_6$.

**5.** Composé selon la revendication 4, qui est le:
7$\beta$-[2-(5-amino-1,2,4-thiadiazol-3-yl)-2-méthoxyiminoacétamido]-3-(3-amino-2-méthyl-1-pyrazolio)méthyl-3-céphème-4-carboxylate (isomère syn).

**6.** Procédé pour préparer un composé de la formule :

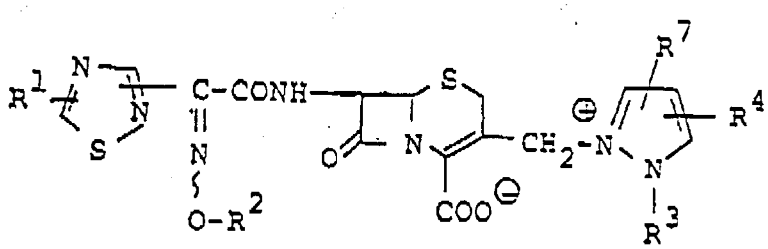

dans laquelle
$R^1$ est un groupe amino ou amino protégé,
$R^2$ est un groupe alkyle en $C_1$ à $C_6$ ou alcényle en $C_1$ à $C_6$,
$R^3$ est un groupe alkyle en $C_1$ à $C_6$, alkyl (en $C_1$ à $C_6$)hydroxy, ou alkyl(en $C_1$ à $C_6$)hydroxy protégé,
$R^4$ est un groupe amino, amino protégé, alkyl(en $C_1$ à $C_6$)amino, alkyl(en $C_1$ à $C_6$)amino protégé, alkyl(en $C_1$ à $C_6$)amino carboxy, N-[alkyl(en $C_1$ à $C_6$)carboxy protégé]amino et
$R^7$ est un atome d'hydrogène ou un groupe alkyle en $C_1$ à $C_6$,
ou un de ses sels,
qui comprend :

(1) le fait de mettre à réagir un composé de la formule :

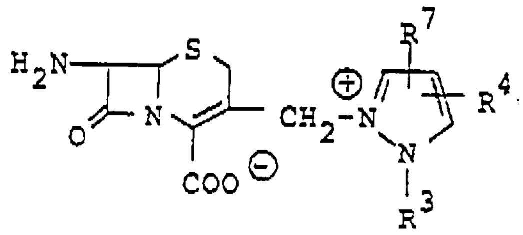

dans laquelle $R^3$, $R^4$ et $R^7$ sont chacun tels que définis ci-dessus, ou son dérivé réactif au groupe amino ou un de ses sels, avec un composé de la formule :

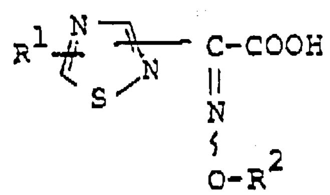

dans laquelle $R^1$ et $R^2$ sont chacun tels que définis ci-dessus, ou avec son dérivé réactif au groupe carboxy ou un de ses sels, pour donner un composé de la formule:

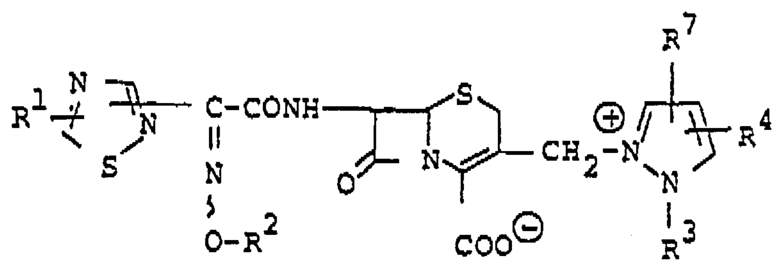

dans laquelle

$R^1$, $R^2$, $R^3$, $R^4$ et $R^7$ sont chacun tels que définis ci-dessus,

ou un de ses sels, ou

(2) le fait de soumettre un composé de la formule :

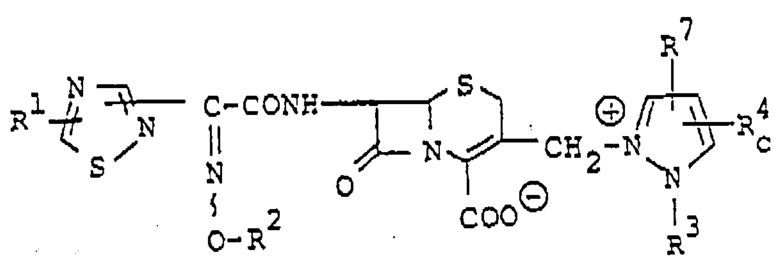

dans laquelle

$R^1$, $R^2$, $R^3$ et $R^7$ sont chacuns tels que définis ci-dessus,

$R^4_c$ est un groupe N-[(alkyl en $C_1$ à $C_6$)carboxy protégé]amino,

ou un de ses sels, à une réaction d'élimination du groupe protecteur du groupe carboxy pour donner un composé de la formule :

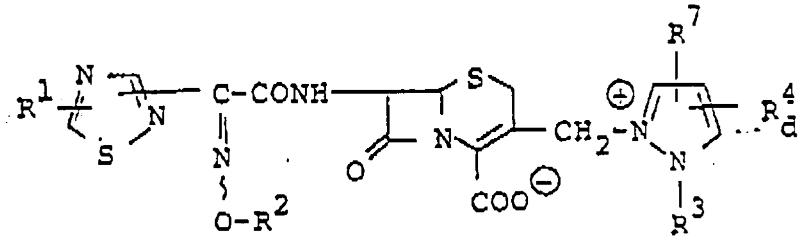

dans laquelle

$R^1$, $R^2$, $R^3$ et $R^7$ sont chacun tels que définis ci-dessus,

$R^4_d$ est un groupe alkyl(en $C_1$ à $C_6$) amino carboxy ou un de ses sels, ou

(3) le fait de soumettre un composé de la formule :

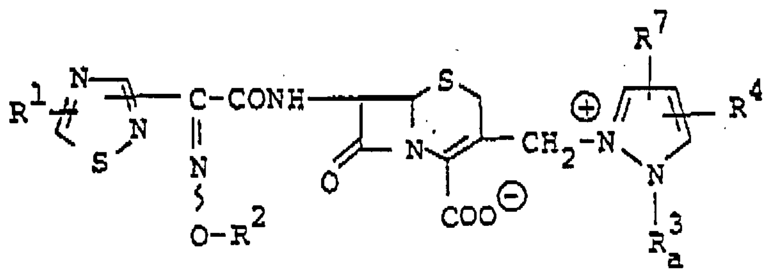

dans laquelle

$R^1$, $R^2$, $R^4$ et $R^7$ sont chacun tels que définis ci-dessus,

$R^3_a$ est un groupe alkyl(en $C_1$ à $C_6$) hydroxy protégé,

ou un de ses sels, à une réaction d'élimination du groupe protecteur du groupe hydroxy pour donner un composé de la formule :

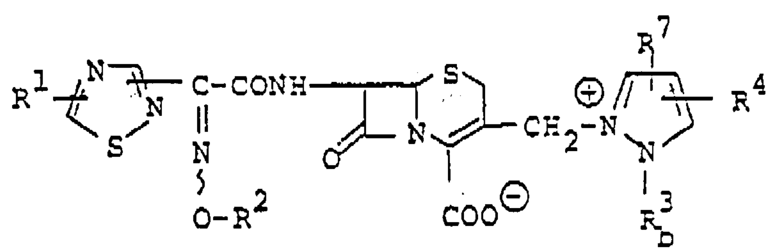

dans laquelle

$R^1$, $R^2$, $R^4$ et $R^7$ sont chacun tels que définis ci-dessus,

$R^3_b$ est un groupe alkyl(en $C_1$ à $C_6$) hydroxy,

ou un de ses sels.

**7.** Composition pharmaceutique qui comprend, comme ingrédient actif, un composé de la revendication 1 ou un de ses sels pharmaceutiquement acceptables en mélange avec des supports pharmaceutiquement acceptables.

**8.** Composé de la revendication 1, ou son sel pharmaceutiquement acceptable pour emploi comme médicament.

**9.** Composé de la revendication 1 ou un de ses sels pharmaceutiquement acceptables pour emploi comme agent antimicrobien.

**10.** Utilisation d'un composé de la revendication 1 ou d'un de ses sels pharmaceutiquement acceptables pour la fabrication d'un médicament pour traiter les maladies infectieuses.

**Revendication pour l'Etat contractant suivant : ES**

**1.** Procédé pour préparer un composé de la formule :

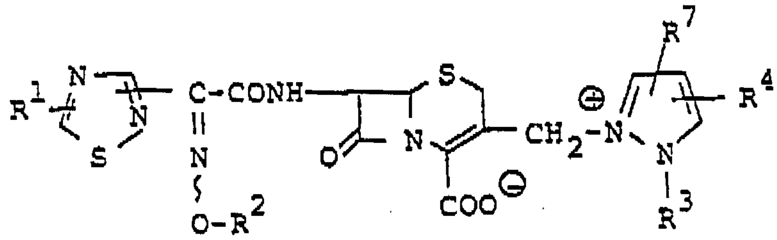

dans laquelle

$R^1$ est un groupe amino ou amino protégé,

$R^2$ est un groupe alkyle en $C_1$ à $C_6$ ou alcényle en $C_1$ à $C_6$,

$R^3$ est un groupe alkyle en $C_1$ à $C_6$, alkyl (en $C_1$ à $C_6$)hydroxy, ou alkyl (en $C_1$ à $C_6$)hydroxy protégé,

$R^4$ est un groupe amino, amino protégé, alkyl(en $C_1$ à $C_6$)amino, alkyl(en $C_1$ à $C_6$)amino protégé, alkyl(en $C_1$ à $C_6$)amino carboxy, N-[alkyl(en $C_1$ à $C_6$)carboxy protégé]amino et

$R^7$ est un atome d'hydrogène ou un groupe alkyle en $C_1$ à $C_6$,

ou un de ses sels,

qui comprend :

(1) le fait de mettre à réagir un composé de la formule :

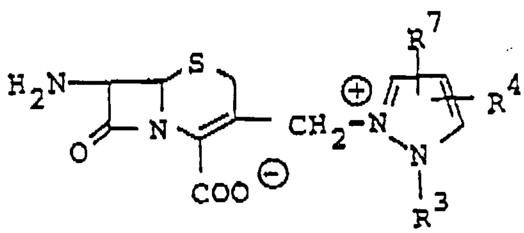

dans laquelle $R^3$, $R^4$ et $R^7$ sont chacun tels que définis ci-dessus, ou son dérivé réactif au groupe amino ou un de ses sels, avec un composé de la formule :

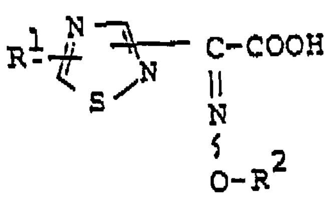

dans laquelle $R^1$ et $R^2$ sont chacun tels que définis ci-dessus, ou son dérivé réactif au groupe carboxy ou un de ses sels, pour donner un-composé de la formule:

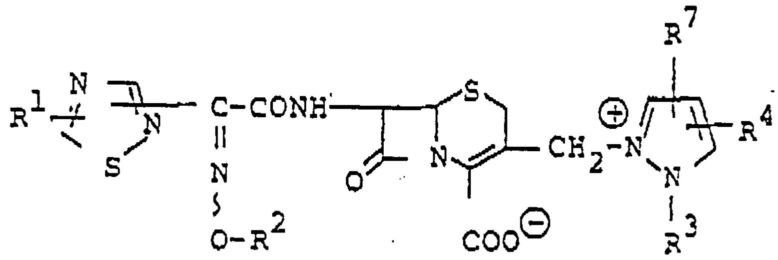

dans laquelle

$R^1$, $R^2$, $R^3$, $R^4$ et $R^7$ sont chacun tels que définis ci-dessus,

ou un de ses sels, ou

(2) le fait de soumettre un composé de la formule :

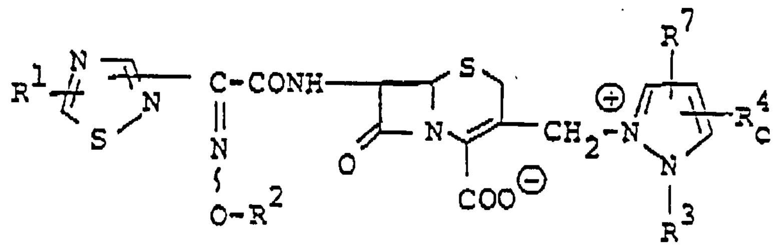

dans laquelle

$R^1$, $R^2$, $R^3$ et $R^7$ sont chacuns tels que définis ci-dessus,

$R^4_c$ est un groupe N-[(alkyl en $C_1$ à $C_6$)carboxy protégé]amino,

ou un de ses sels, à une réaction d'élimination du groupe protecteur du groupe carboxy, afin d'obtenir un composé de la formule :

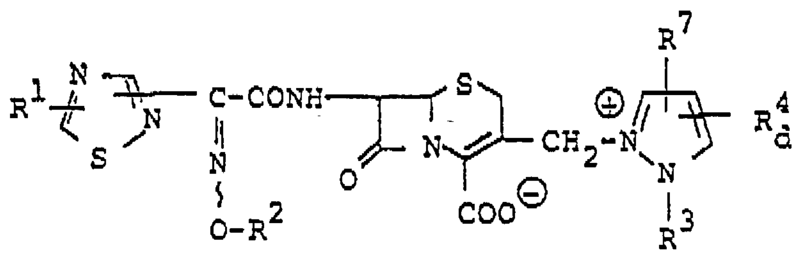

dans laquelle

$R^1$, $R^2$, $R^3$ et $R^7$ sont chacun tels que définis ci-dessus,

$R^4_d$ est un groupe alkyl(en $C_1$ à $C_6$) amino carboxy,

ou un de ses sels, ou

(3) le fait de soumettre un composé de la formule :

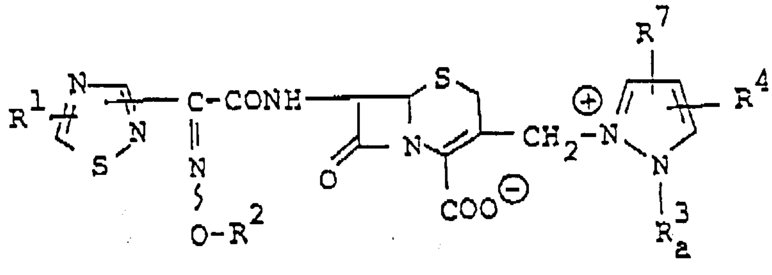

dans laquelle
$R^1$, $R^2$, $R^4$ et $R^7$ sont chacun tels que définis ci-dessus,
$R^3_a$ est un groupe alkyl(en $C_1$ à $C_6$) hydroxy protégé,
ou un de ses sels, à une réaction d'élimination du groupe protecteur du groupe hydroxy afin de donner un composé de la formule :

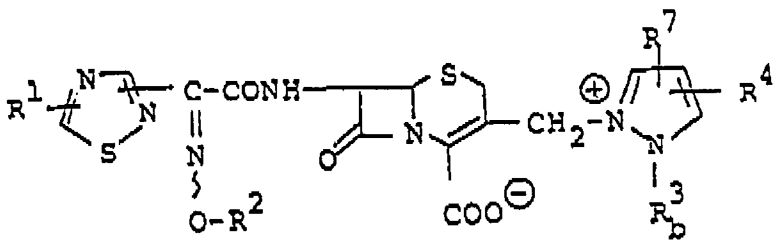

dans laquelle
$R^1$, $R^2$, $R^4$ et $R^7$ sont chacun tels que définis ci-dessus,
$R^3_b$ est un groupe alkyl(en $C_1$ à $C_6$) hydroxy,
ou un de ses sels.

**Revendications pour l'Etat contractant suivant : GR**

**1.** Procédé pour préparer un composé de la formule :

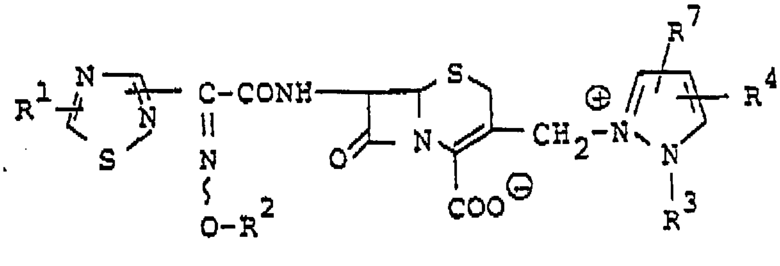

dans laquelle
$R^1$ est un groupe amino ou amino protégé,
$R^2$ est un groupe alkyle en $C_1$ à $C_6$ ou alcényle en $C_1$ à $C_6$,
$R^3$ est un groupe alkyle en $C_1$ à $C_6$, alkyl (en $C_1$ à $C_6$)hydroxy, ou alkyl (en $C_1$ à $C_6$)hydroxy protégé,
$R^4$ est un groupe amino, amino protégé, alkyl(en $C_1$ à $C_6$)amino, alkyl(en $C_1$ à $C_6$)amino protégé, alkyl(en $C_1$ à $C_6$)amino carboxy, N-[alkyl(en $C_1$ à $C_6$)carboxy protégé]amino et
$R^7$ est un atome d'hydrogène ou un groupe alkyle en $C_1$ à $C_6$,
ou un de ses sels,

qui comprend :

(1) le fait de mettre à réagir un composé de la formule :

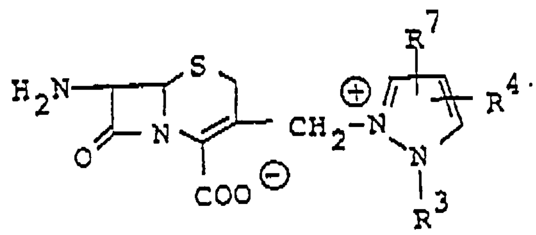

dans laquelle $R^3$, $R^4$ et $R^7$ sont chacun tels que définis ci-dessus, ou son dérivé réactif au groupe amino, ou un de ses sels, avec un composé de la formule :

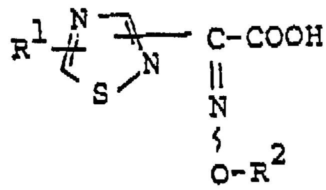

dans laquelle $R^1$ et $R^2$ sont chacun tels que définis ci-dessus, ou son dérivé réactif au groupe carboxy ou un de ses sels, pour donner un composé de la formule:

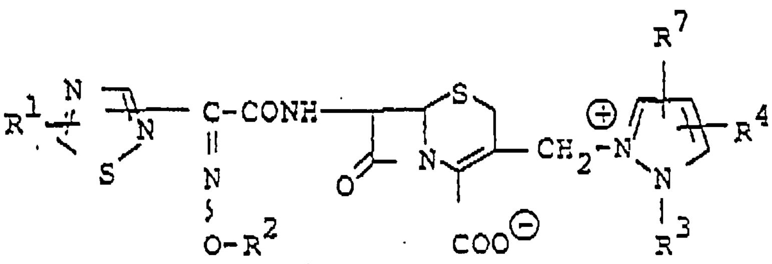

dans laquelle

$R^1$, $R^2$, $R^3$, $R^4$ et $R^7$ sont chacun tels que définis ci-dessus,

ou un de ses sels, ou

(2) le fait de soumettre un composé de la formule :

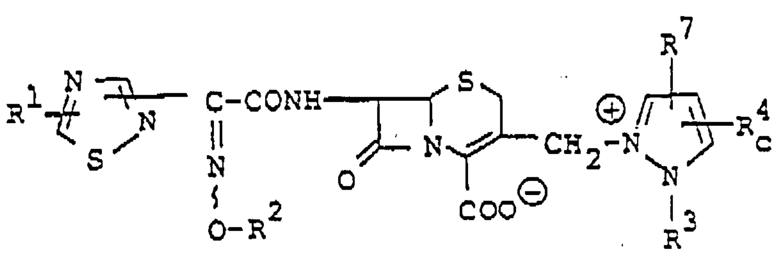

dans laquelle

$R^1$, $R^2$, $R^3$ et $R^7$ sont chacuns tels que définis ci-dessus,

$R^4_c$ est un groupe N-[(alkyl en $C_1$ à $C_6$)carboxy protégé]amino,

ou un de ses sels, à une réaction d'élimination du groupe protecteur du groupe carboxy pour donner un composé de la formule :

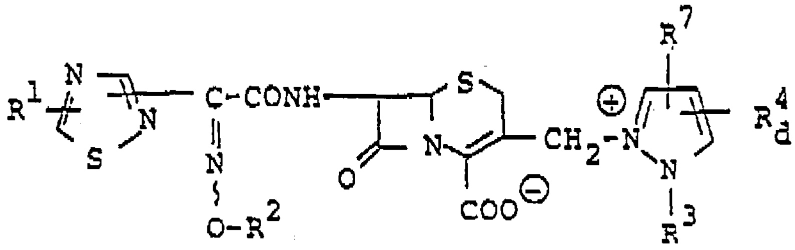

dans laquelle

$R^1$, $R^2$, $R^3$ et $R^7$ sont chacun tels que définis ci-dessus,

$R^4_d$ est un groupe alkyl(en $C_1$ à $C_6$) amino carboxy,

ou un de ses sels, ou

(3) les fait de soumettre un composé de la formule :

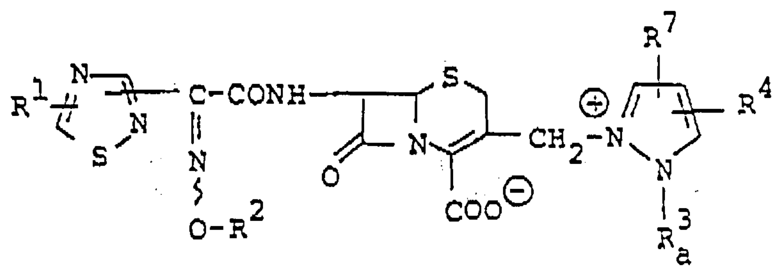

dans laquelle

$R^1$, $R^2$, $R^4$ et $R^7$ sont chacun tels que définis ci-dessus,

$R^3_a$ est un groupe alkyl(en $C_1$ à $C_6$) hydroxy protégé,

ou un de ses sels, à une réaction d'élimination du groupe protecteur du groupe hydroxy pour donner un composé de la formule :

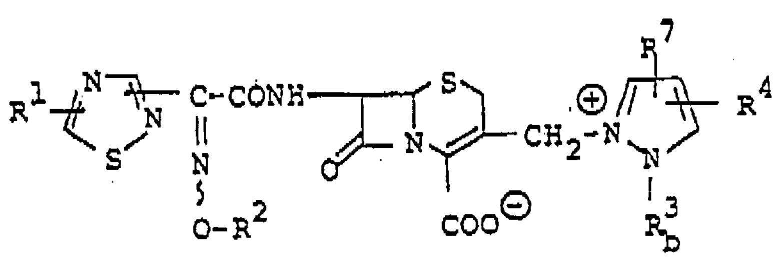

dans laquelle

$R^1$, $R^2$, $R^4$ et $R^7$ sont chacun tels que définis ci-dessus,

$R^3_b$ est un groupe alkyl(en $C_1$ à $C_6$) hydroxy,

ou un de ses sels.

2. Modification du procédé de la revendication 1 qui est caractérisée en ce qu'on amène un composé préparé par le procédé de la revendication 1 à une forme pharmaceutiquement acceptable par mélange ou présentation dudit composé avec des supports pharmaceutiquement acceptables.